# EUROPÄISCHE PATENTANMELDUNG

(11) **EP 0 439 130 A1**
(43) Veröffentlichungstag der Anmeldung: **31.07.1991**
(21) Anmeldenummer: 91100797.9
(22) Anmeldetag: 23.01.1991
(51) Int. Cl.: A01N 43/64, A01N 35/04, A01N 35/02, C07D 251/04, C08G 12/26

(54) **Bakterizide, virizide und fungizide Mischung**

(30) Priorität: 23.01.1990 DD 337265; 23.01.1990 DD 337264; 23.01.1990 DD 337263; 23.01.1990 DD 337262; 23.01.1990 DD 337261; 23.01.1990 DD 337259; 23.01.1990 DD 337260
(71) Anmelder: ASV-innovative Chemie GmbH, D-06733 Bitterfeld (DE)
(72) Erfinder: Thust, Ulf, Dr., O-4500 Dessau (DE); Kellner, Kurt, Prof. Dr., O-4090 Halle-Neustadt (DE); Naumann, Jutta, O-7270 Delitzsch (DE); Trautner, Kurt, O-4400 Bitterfeld (DE); Fieseler, Christine, O-4415 Zörbig (DE); Pfeiffer, Hans-Dieter, Dr., O-4440 Wolfen (DE); Wigert, Heinz, Dr., O-9659 Markneukirchen (DE)

(57) **Zusammenfassung**

Die Erfindung betrifft eine bakterizide, virizide und fungizide Mischung, die neben üblichen Hilfs- und Trägerstoffen Wirkstoffe enthält, die durch Umsetzung von N,N',N"-1,3,5-Tris-(2-hydroxyethyl)hexahydrotriazin oder äquimolaren Mengen Formaldehyd und Ethanolamin mit aliphatischen oder aromatischen Mono- oder Dialdehyden entstehen.

Die neuen Wirkstoffe eignen sich sehr gut für eine Anwendung als Desinfektionsmittel, Konservierungsmittel, Pflanzenschutzmittel sowie als Zusätze zu Flüssigwaschmitteln.

Der große Vorteil der erfindungsgemäßen Mittel besteht in einer großen Anwenderfreundlichkeit und Verträglichkeit, da keine freien Aldehyde nachgewiesen werden können.

## Beschreibung

Die Erfindung betrifft eine neue bakterizide, virizide und fungizide Mischung, die zur Anwendung in der Desinfektion, zur Konservierung, zum Pflanzenschutz und zur Schädlingsbekämpfung geeignet ist. Ebenso bestehen gute Anwendungsmöglichkeiten als Zusatz für Flüssigwaschmittel und für Kühlschmiermittel in der Metallbearbeitung.

1,3,5-Tris-(2-hydroxyethyl)hexahydrotriazin wird durch Kondensation von Formaldehyd und Ethanolamin gebildet (DE-AS 1 148 706; DE-OS 1 812 054). Es ist als Wirkstoff in Konservierungsmitteln bekannt (DD-PS 248 500; RD-PS 66 015; JP-PS 82 36 191) und wurde auch als Bestandteil von Desinfektionsmitteln beansprucht (JP-PS 81 57 874; US-PS 3 981 998). Häufig wird es in Kombination mit anderen Wirkstoffen eingesetzt (US-PS 4 352 744; US-PS 3 981 998; US-PS 4 071 628; DE-OS 23 37 755).

Allgemein bekannt sind Umsetzungen von Aldehyden mit Alkoholen zu Semiacetalen, die unter Protonenkatalyse mit weiterem Alkohol zu Acetalen reagieren. Die Semiacetale liegen stets im Gleichgewicht mit den entsprechenden Aldehyden vor (Houben-Weyl, 6/3, 214). Gleiches trifft für cyclische Semiacetale zu, die entsprechenden CO-Hydroxyaldehyde liegen in Abhängigkeit von der Kettenlänge im erheblichen Anteil im Gleichgewicht vor (Houben-Weyl 6/4, 335 ff, 1966).

Dialdehyde stehen mit ihren cyclischen Halbacetalen in wäßriger oder alkoholischer Lösung ebenfalls im Gleichgewicht (Houben-Weyl, E3, S. 610, 1983).

Desinfektionsmitteln auf Aldehydbasis, insbesondere mit Dialdehyden, wird durch Kombination mit Alkoholen eine größere Stabilität und Erhalt der Wirksamkeit zugeschrieben. (US-PS 3 983 252)

Unter alkalischen Bedingungen neigen Dialdehyde zur Polymerisation. Durch Zusatz von Diolen lassen sich diese Dialdehyd-Lösungen mehr oder weniger gut stabilisieren. Als bislang zweckmäßigste Einsatzform werden Zweikomponentensysteme angesehen, die vor Gebrauch mit alkalisierenden Zusätze versehen werden (US-PS 3 983 252).

Mitunter wird der Dialdehyd in einer lagerstabilen Mischung oder Vorstufe eingesetzt, aus der er durch Zugabe von Wasser in Gegenwart saurer Katalysatoren im Moment der Anwendung freigesetzt wird (US-PS 4 448 977, US-PS 4 244 876).

Umsetzungen von 1,3,5-Tris-(2-hydroxyethyl)hexahydrotriazin mit Aldehyden sind in der Literatur noch nicht beschrieben.

Ziel der Erfindung ist die Herstellung von formaldehydfreien, stabilen und wasserlöslichen Verbindungen sowie deren Verwendung als antimikrobielle Zusätze für verschiedene Anwendungsgebiete.

Die Aufgabe der Erfindung besteht darin, acetalartige Verbindungen herzustellen, die sowohl den technischen Anforderungen wie Lagerstabilität, Wasserlöslichkeit und Formaldehydfreiheit, als auch den Anwendungsforderungen als biologisch-wirksame Substanzen, genügen.

Diese Aufgabe wird erfindungsgemäß dadurch gelöst, daß die virizide, bakterizide und fungizide Mischung neben üblichen Zusatzstoffen aus acetalartigen Verbindungen besteht, die bei der Umsetzung von N,N',N''-1,3,5-Tris-(2-hydroxyethyl)-hexahydrotriazin (im folgenden als HHT bezeichnet) oder von äquimolaren Mengen Formaldehyd und Ethanolamin mit aliphatischen oder aromatischen Mono- oder Dialdehyden bei 10 bis 100 °C entstehen, wobei das Äquivalenzverhältnis der OH-Gruppen zu den Aldehydgruppen 10 : 1 bis 9 beträgt. Diese so hergestellten Ethanolaminacetale (im folgenden als EA-Acetale bezeichnet) sind aldehydgruppenfrei, wasserlöslich und lagerstabil.

Als aliphatische oder aromatische Mono- oder Dialdehyde werden vorzugsweise solche mit 2 bis 8 C-Atomen, beispielsweise Glutaraldehyd, Malondialdehyd, n- oder iso-Butyraldehyd, n- oder iso-Butyraldehyd, Propionaldehyd, Acetaldehyd, Chloral, Benzaldehyd oder Vanillin, verwendet.

Als Lösungsmittel werden vorzugsweise Wasser, Alkohole, Ether, chlorierte Kohlenwasserstoffe oder Aromaten verwendet. Diese Können nach erfolgter Umsetzung wieder entfernt werden. Nach der Umsetzung kann durch Zugabe organischer oder anorganischer Säuren ein pH-Wert von 4 bis 12 eingestellt werden. Die EA-Acetale oder ihre Lösungen sind in diesem pH-Bereich ohne Stabilisatoren mehr als 9 Monate lagerstabil. Für das diskontinuierliche Verfahren wird vorteilhaft HHT vorgelegt und der Aldehyd in Substanz oder in Lösung zugegeben. Es erweist sich als günstig, bei exothermen Reaktionen das Reaktionsgemisch durch Kühlung auf Temperaturen unter 100 °C zu halten. Die Umsetzungen erfolgen vollständig, die erhaltenen Produkte weisen keine Aldehydfunktion mehr auf, die typischen Aldehydgerüche treten nicht mehr auf. In den Wirkstofflösungen (EA-Acetale) könnten mittels Dünnschichtchromatographie, IR-, NMR- und UV-Spektroskopie keine Aldehydfunktionen nachgewiesen werden. Die erfindungsgemäß erhaltenen EA-Acetale enthalten in allen Fällen freies 1,3,5-Tris-(2-hydroxyethyl)hexahydrotriazin.

Die Aldehydfreiheit wurde durch die gefundene überraschend hohe Hautverträglichkeit und durch das Ausbleiben von Sensibilisierungserscheinungen mit Allergiefolgen bei Mensch und Tier indirekt bestätigt. Die Aldehydfreiheit war auf der Grundlage des bekannten Standes der Technik völlig überraschend, da für acetalartige Verbindungen das Vorliegen freier Aldehyde erwartet werden mußte.

Die erfindungsgemäße neue bakterizide, virizide und fungizide Mischung kann Bestandteil neuer Desinfektionsmittel, Konservierungsmittel und Pflanzenschutzmittel zur Bekämpfung von Bakterien, Pilzen oder Viren sein. Ebenso ist eine Anwendung in Flüssigwaschmitteln sowie als Zusatz für Kühlschmiermittel in der Metallbearbeitung möglich.

Erfindungsgemäß enthalten die neuen Desinfektions-, Konservierungs-und Pflanzenschutzmittel sowie die neuen hygienischen Flüssigwaschmittel und Kühlschmiermittel neben üblichen Roh-, Hilfs- und Trägerstoffen, HHT und bekannten mikrobiziden quartären Ammoniumsalzen als neue Wirkstoffe EA-Acetale im Gewichtsverhältnis von EA-Acetalen zu quartären Ammoniumsalzen zu HHT von 0,5 bis 20 : 0,1 bis 10 : 1. Zusätzlich können je nach Einsatzfall Kombinationen mit weiteren Wirkstoffen vorteilhaft sein. Die erfindungsgemäßen Mittel enthalten vorwiegend 10 bis 60 % Wirkstoffen und zeigen je nach Einsatzfall ab 0,01 % biologische Wirkung.

Für den praktischen Einsatz werden empfohlen für
Desinfektionszwecke 0,3 - 5 %
Konservierungszwecke 0,1 - 3 %, vorzugsweise 0,1 - 1 %
den Pflanzenschutz in Hydrokulturen 0,01 - 2 %
Desinfektion in Gewächshäusern 0,5 - 5 %
Vernebelungstechnik bis 15 %
Kühlschmiermittel 3 - 10 %
Flüssigwaschmittel 5 - 15 %.

EA-Acetale mit Glutardialdehyd sind besonders empfehlenswert. Die neuen EA-Acetale und erfindungsgemäßen Mittel auf dieser Basis zeichnen sich durch besonders günstige Anwenderfreundlichkeit hinsichtlich Hautverträglichkeit, geringer Toxizität, Korrosivität und Geruchsbelästigung aus. Dazu kommt Wasserlöslichkeit, gute Lagerstabilität bei pH 7 - 13, gute Pflanzen- und Materialverträglichkeit. Dadurch werden die Arbeitsbedingungen für die Anwender vor allem hinsichtlich der Risiken für die Gesundheit entscheidend verbessert.

### Ausführungsbeispiele

### 1. Beispiel: Herstellung

1.1. In 20 ml Methanol werden 10,6 g (0,05 Mol) HHT /1,3,5-Tris-(2-hydroxyethyl)-hexahydrotriazin/ gelöst und unter Rühren mit 7,1 g (0,05 Mol) Isobutyraldehyd versetzt. Nach 30 Minuten wird das Lösungsmittel im Vakuum entfernt, es verbleiben 17,4 g Produkt, was einer 98 %igen Ausbeute entspricht. Spektroskopische Untersuchungen zeigen die 100 %ige Umwandlung des Aldehyds in Acetal- und Semiacetalform. Dünnschichtchromatographisch wurde freies 1,3,5-Tris-(2-hydroxy-ethyl)-hexahydrotriazin festgestellt.
1.2. Zu 21,5 g (0,1 Mol) HHT in 20 ml Chloroform werden unter Kühlung 13,5 g (0,1 MDl) Chloral, gelöst in 20 ml Chloroform, gegeben. Nach 1 Stunde wird das Produkt vom Lösungsmittel befreit, es resultieren 33,7 0 Produkt (96,3 %). Spektroskopische Untersuchungen zeigen eine vollständige Umwandlung in acetalartige Verbindungen an.
1.3. Zu 2,57 kg 83 %iger wäßriger Lösung von HHT werden unter Kühlung 1,0 kg einer 50 %igen wäßrigen Lösung von Glutaraldehyd so zugegeben, daß die Temperatur im Reaktionsgefäß 60 °C nicht überschreitet. Nach Abklingen der exothermen Reaktion wird eine Stunde gerührt. Im Reaktionsprodukt ist danach kein freier Aldehyd nachweisbar. Die Lösung ist 74 %ig an Produkt und kann direkt eingesetzt werden.
1.4. Zu 300 g Formalin (30 %ig) werden nacheinander 177 g Ethanolamin und 100 g 50 %ige wäßrige Lösung von Glutaraldehyd derart gegeben, daß die Temperatur in der Mischung max. 70 °C beträgt. 60 Minuten nach vollständiger Zugabe sind im Reaktionsprodukt kein Formaldehyd und kein Glutaraldehyd nachweisbar. Die Lösung ist 45,5 %ig an Wirkstoff und kann direkt eingesetzt werden.
1.5. 10 g HHT werden in 10 ml Methanol gelöst und zu 1,8 g Salicylaldehyd in 100 ml Methanol gegeben. Nach 1 Stunde bei Zimmertemperatur wird spektroskopisch eine vollständige Umsetzung beobachtet. Die Entfernung des Lösungsmittels im Vakuum liefert 11,6 g (98 %) Wirkstoff, der gut wasserlöslich ist.

### 2. Beispiel: Verwendete Abkürzungen für Vergleichsmuster und erfindungsgemäße Mischungen

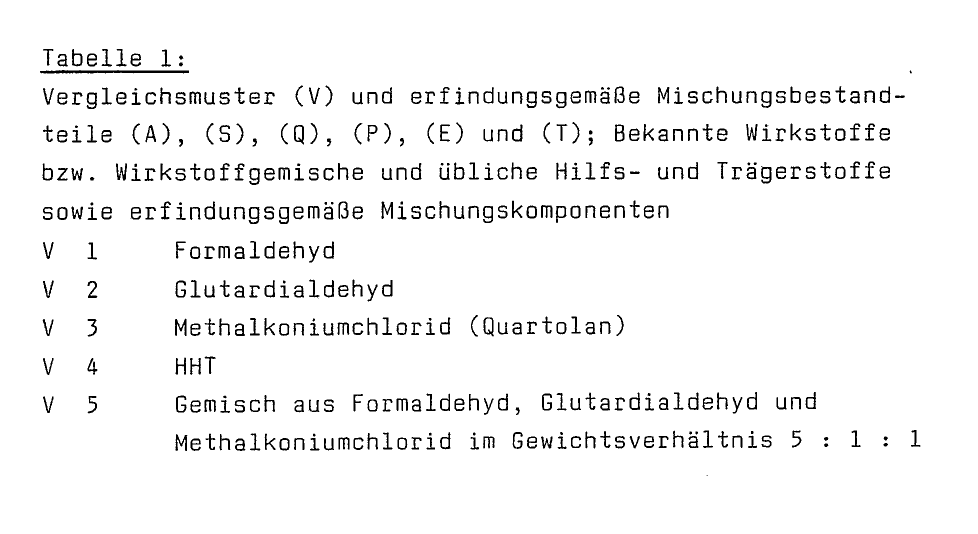

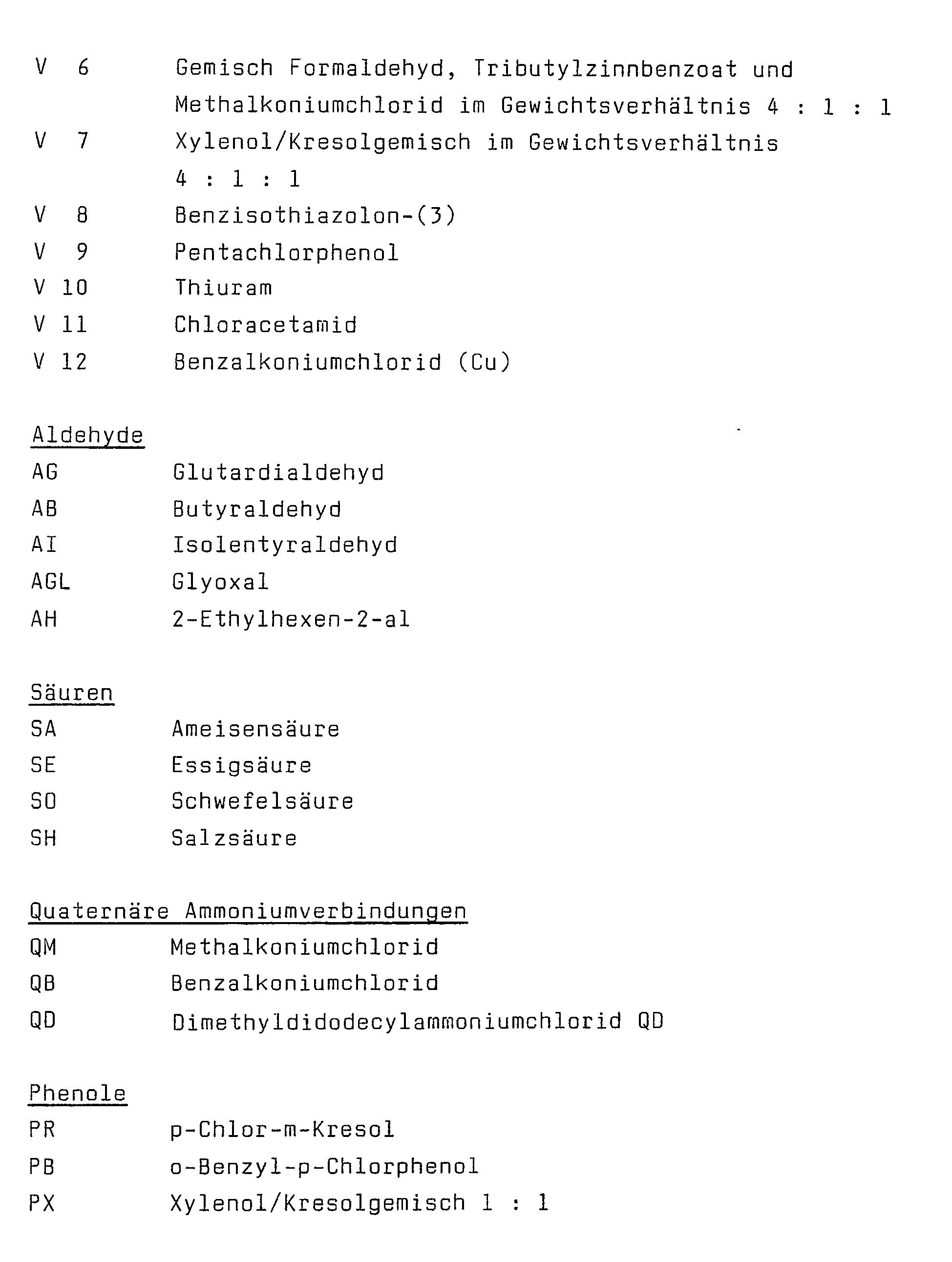

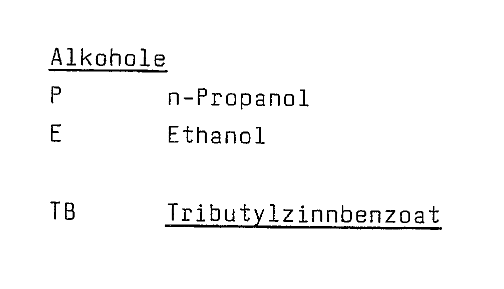

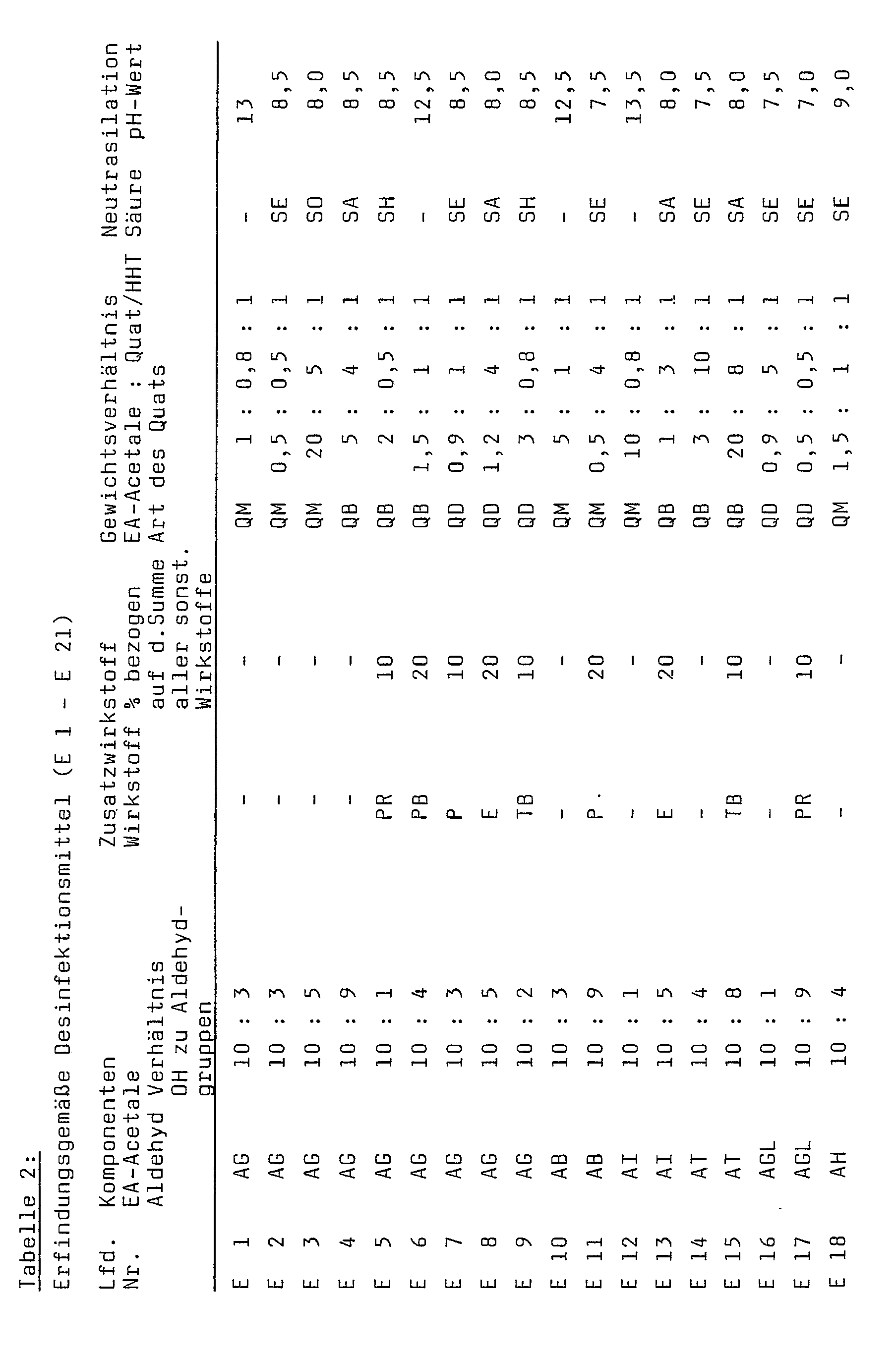

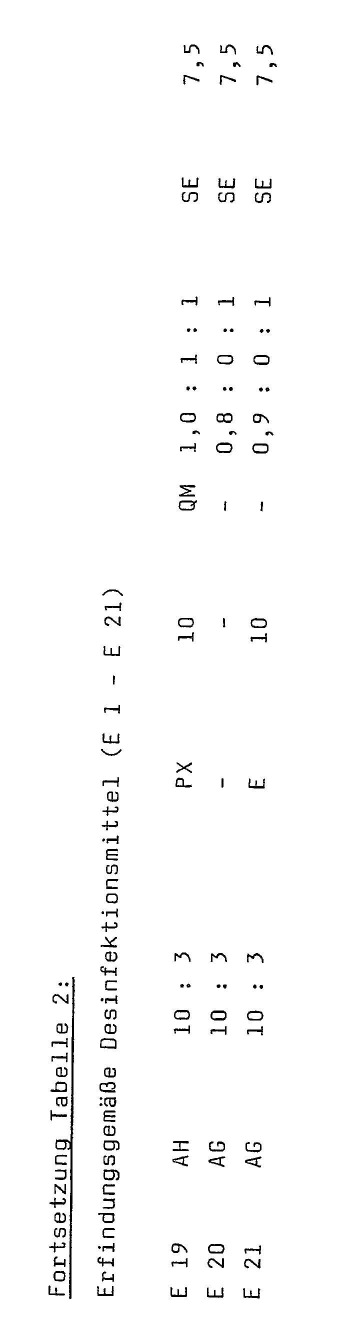

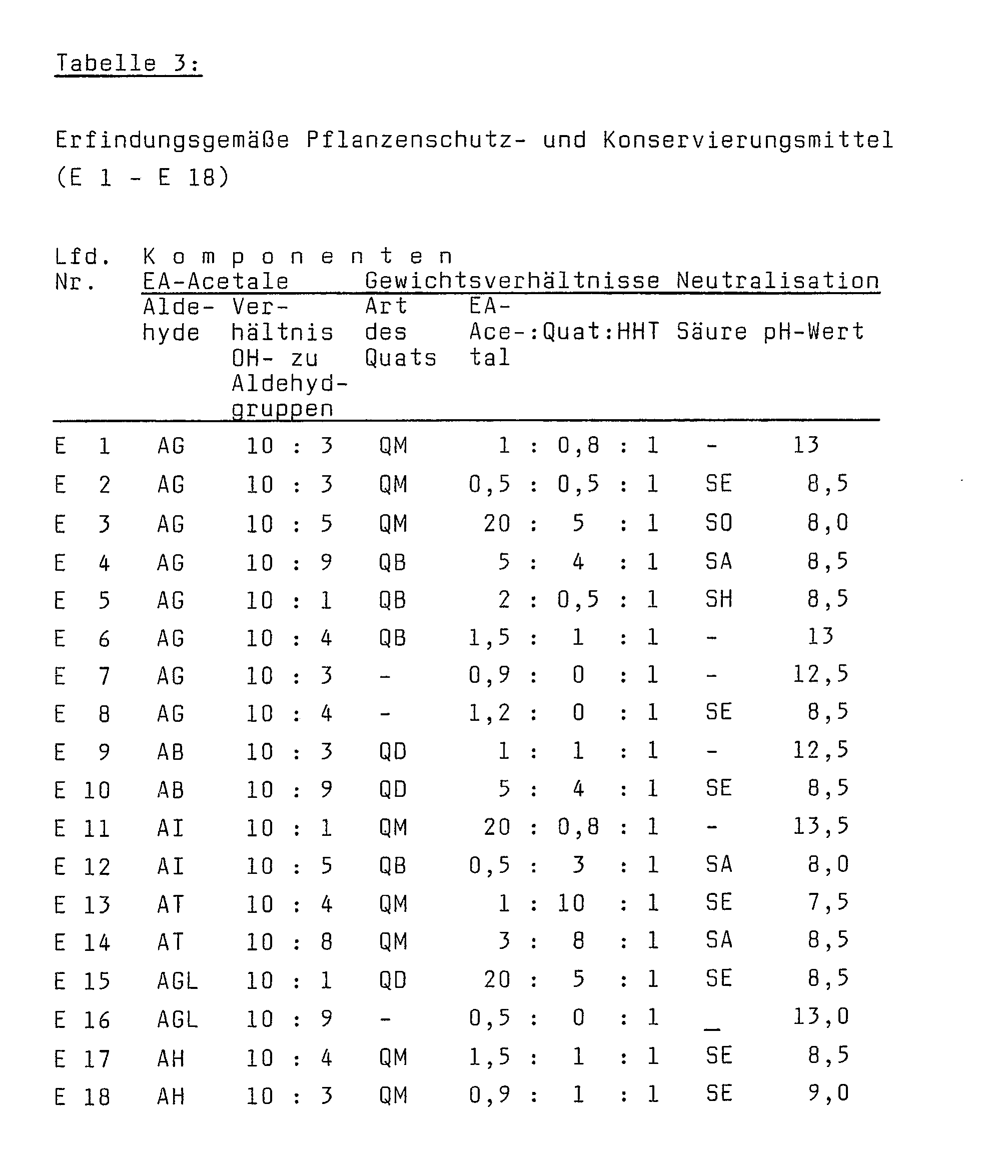

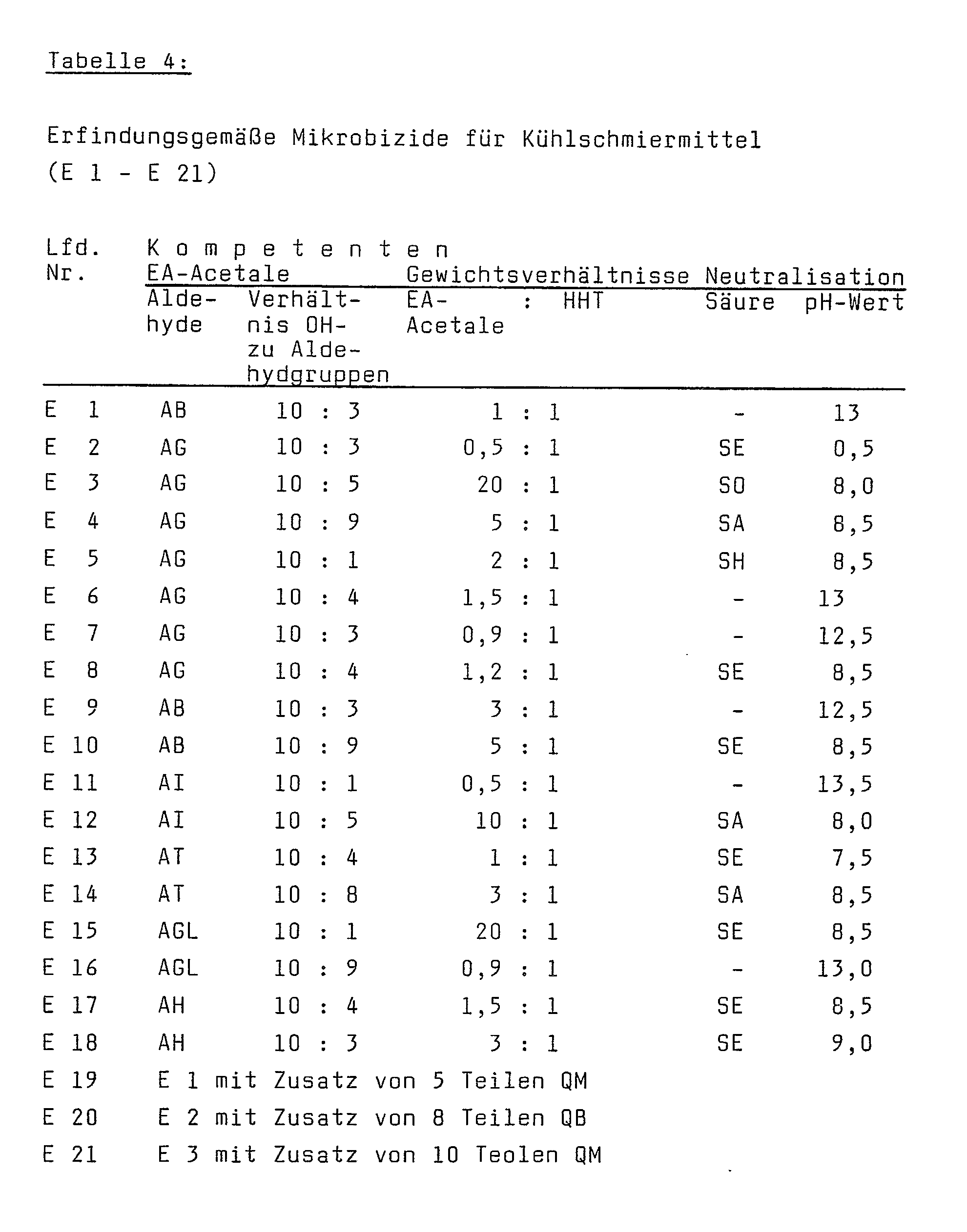

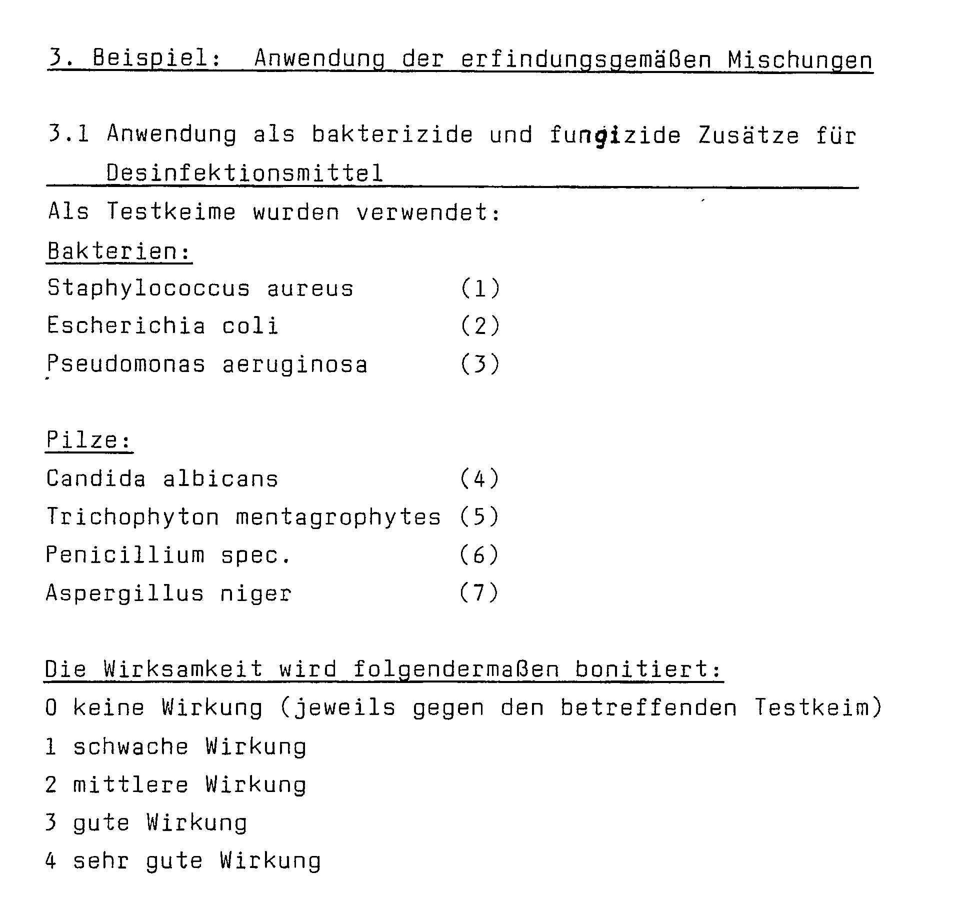

### Testmethoden

### 1. Suspensionstest:

Zu 10 ml Prüflösung unterschiedlicher Wirkstoffkonzentrationen wird je 0,1 ml einer standardisierten Bouillonkultur der Testkeime gegeben und das Ganze gut vermischt. Nach Einwirkungszeiten von 2 bis 120 min wird jeweils eine Platinöse Gemisch entnommen und in 10 ml geeigneter Nährmedien überführt. Den Nährmedien werden zur Inaktivierung der Wirkstoffe spezifisch wirkende Hemmstoffe zugesetzt. Anschließend wird für Testbakterien 5 Tage bei 37 °C, für Testpilze 3 Wochen bei 22 °C bebrütet.

### Flächendesinfektion (praxisanaloger Test)

### Für Bakterien:

Auf sterilisierte Keimträger aus lackiertem Holz und PVC in der Größe von 2 cm x 5 cm werden je 0,1 ml einer standardisierten Testkeimsuspension aufgetragen und mit einem Glasspatel gleichmäßiger verteilt. Nach 30 minütigem Antrocknen bei 20 °C werden 0,1 ml der Prüflösung aufgetragen und ebenfalls gleichmäßig verteilt. Nach Einwirkungszeiten von 1, 2, 3 und 4 h werden die Testflächen auf geeignete feste Nährmedien aufgelegt und für 2 h dort belassen. Die Nährmedien,denen zur Inaktivierung der Wirkstoffe spezifisch wirkende Inhibitoren zugesetzt wurden, werden 3 Tage bei 37 °C bebrütet.

### Für Pilze:

Sterilisierte Holzstäbchen von 2 cm Länge, 2 mm Breite und 2 mm Dicke werden durch 10 minütiges Einlegen in standardisierte Testpilzsuspension kontaminiert, für 1 h bei 37 °C getrocknet und anschließend 5 min in 10 ml der Prüflösung eingelegt. Danach werden die Holzstäbchen in sterilen Glasgefäßen schräg aufgestellt und verbleiben dort für Einwirkungszeiten von 1, 2, 3 und 4 h. Dann werden 50 % der Hölzchen sofort, 50 % nach einer Zwischenspülung mit sterilem Wasser schräg auf Sabouraud-Glucose-Agar aufgelegt, so daß der untere Rand des Hölzchens dicht oberhalb des Kondenswasserspiegels liegt. Dem Agar werden zur Inaktivierung der Wirkstoffe spezifisch wirkende Inhibitoren zugesetzt. Die Nährmedien werden bis zu 4 Wochen bei 22 °C bebrütet.

### Instrumentendesinfektion (praxisanaloger Test)

Sterilisierte Keimträger werden zur Kontamination 10 min in eine standardisierte Testkeimsuspension eingelegt. Als Keimträger dienen Glasplättchen (2,5 x 1 cm), Plastplättchen (2,5 x 1 cm), Gummischlauch (halbiert, 2 cm) und Metallschrauben (2 cm).

Die kontaminierten Keimträger werden 15 min bei 20 °C getrocknet, danach in jeweils 10 ml Prüflösung eingelegt. Nach Einwirkungszeiten von 15, 30, 45, 60 und 120 min werden sie entnommen, mit sterilem Wasser abgespült und in je 10 ml geeignetes flüssiges Nährmedium überführt, Spülflüssigkeit und Nährmedien enthalten spezifisch wirkende Inhibitoren, um die Wirkstoffe zu inaktivieren. Bebrütet wird für 7 Tage bei 37 °C. Die vergleichenden Prüfungen erfolgten bei gleichen Gesamtwirkstoffkonzentrationen. die Ergebnisse der Prüfungen sind in Tabellenform dargestellt:

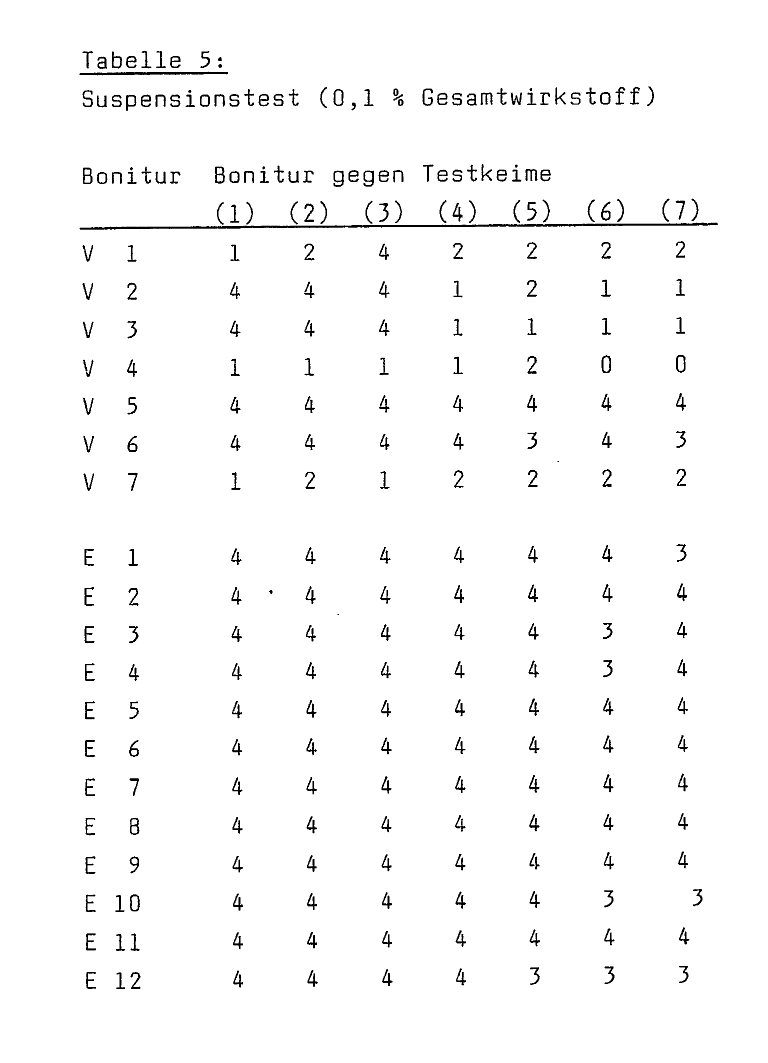

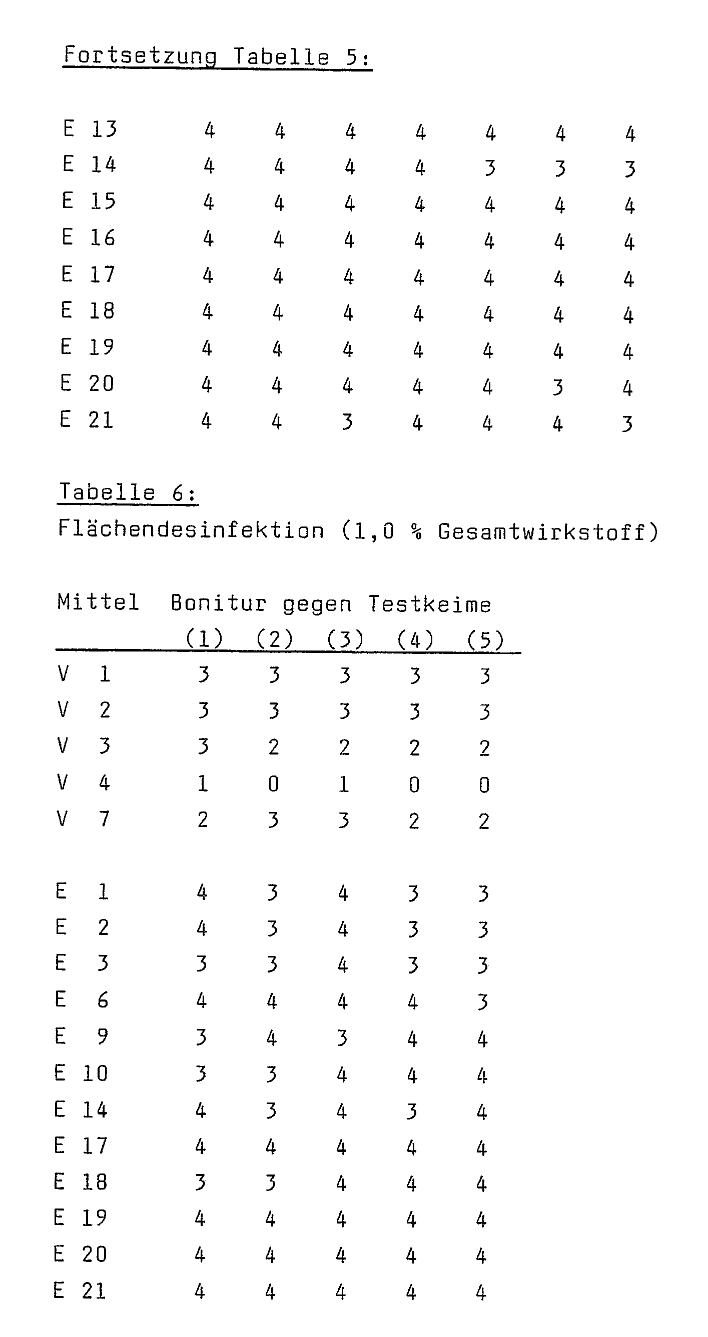

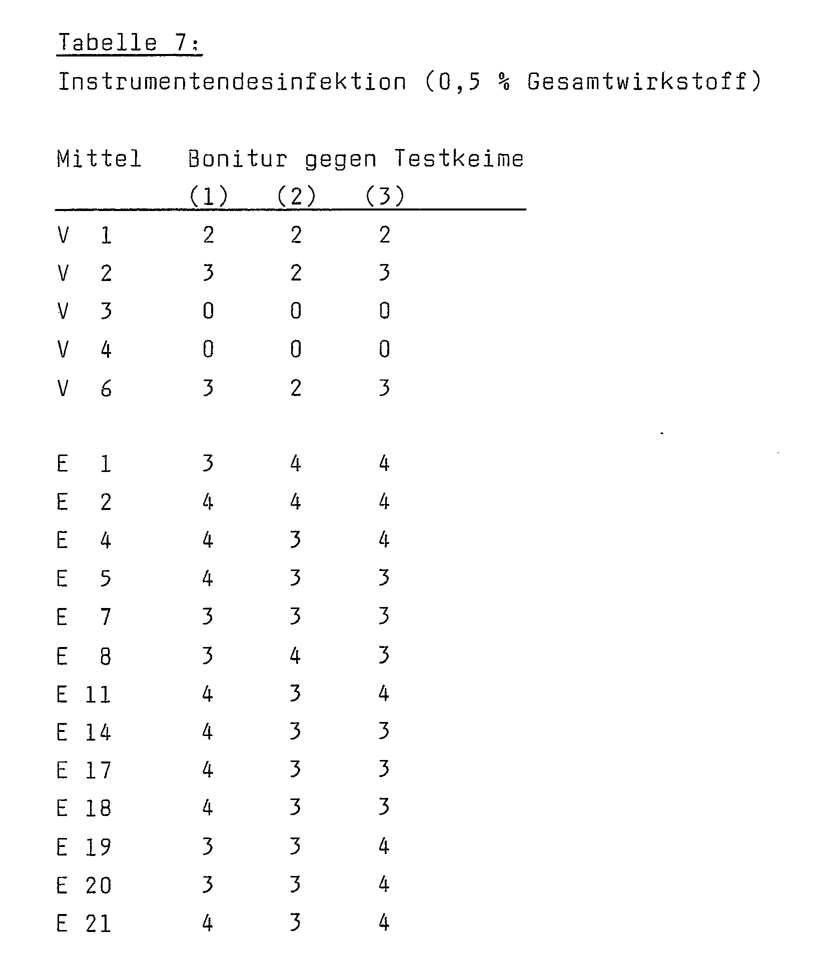

### Virizide Wirksamkeit

### Methodik

Die Prüfung erfolgte gegenüber behüllten Aujetzky-Viren (1) und nicht behüllten Talfan/Teschen-Viren (2) nach einem standardisierten Holzkeimträgermodell bei 20 °C. Der Ausgangstiter wurde nach Antrocknung bestimmt und die Resttiter nach bestimmten Einwirkzeiten ermittelt. Die Ergebnisse wurden boniert:
0 keine Wirksamkeit
1 schwache Wirksamkeit
2 gute Wirksamkeit
3 sehr gute Wirksamkeit

Die Ergebnisse sind in einer Tabelle bei 0,3 % Gesamtwirkstoff dargestellt:

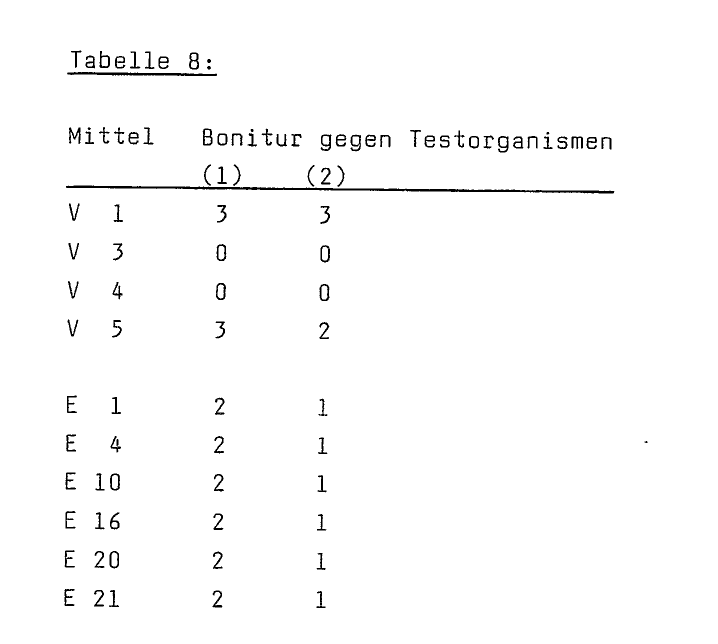

### 3.2. Anwendung als Zusätze für die Konservierung von Kunstharzdispersionen und Haushaltschemikalien

### 3.2.1. Kunstharzdispersionen

### Methodik:

Prüfung auf Keimfreiheit (Test A) Die technischen Dispersionen [Polyvinylacetat, Typ deckweiß (PV, Polyacrylat-Fassadenfarbe (PA), Polyacrylat-Einlaßgrund (EG), im folgenden werden die Abkürzungen verwendet] werden mit dem Konservierungsmittel unter Angabe der Gesamtwirkstoffkonzentration in Masseanteilen in % gemischt. Anschließend werden Proben von 1 ml entnommen und in abgeflammte, mit Sabourand-Nährmedien (1) bzw. mit Thioglykolat-Nährmedien (2) gefüllte Reagenzgläser gegeben. Die Proben mit Kulturmedium (1) werden 10 Tage bei 22 - 24 °C und die mit Kulturmedium (2) 10 Tage bei 30-32 °C im Brutschrank bebrütet. Bonitur:
0 kein Befall
1 geringer Befall
2 starker Befall

Prüfung auf Lagerstabilität (Test B)
Die technischen Dispersionen PV, PA und EG werden mit den Konservierungsmitteln gemischt (Angabe der Gesamtwirkstoffkonzentration), in einem Prüfglas verschlossen und bei Raumtemperatur gelagert. Die Bewertung erfolgt monatlich nach folgender Bonitur:
0 kein Befall
1 geringer Befall
2 starker Befall

Prüfung auf Filmstabilität (Test C)
Anstrichträger aus Filterpapier werden mit der zu prüfenden konservierten technischen Dispersion beschichtet. Die getrockneten Filme werden in Petrischalen auf Nähragar gelegt und mit Sporensuspensionen von Penicillium funiculosum (1), Pullularia pullulans (2) und Aspergillus niger (3) besprüht. Danach erfolgt eine 4wöchige Bebrütung im Brutschrank bei 25 °C mit wöchentlicher Bonitur. Bonitur 0 kein Befall
1 geringer Befall
2 starker Befall

Die Ergebnisse der Prüfungen sind nachfolgend in Tabellenform dargestellt.

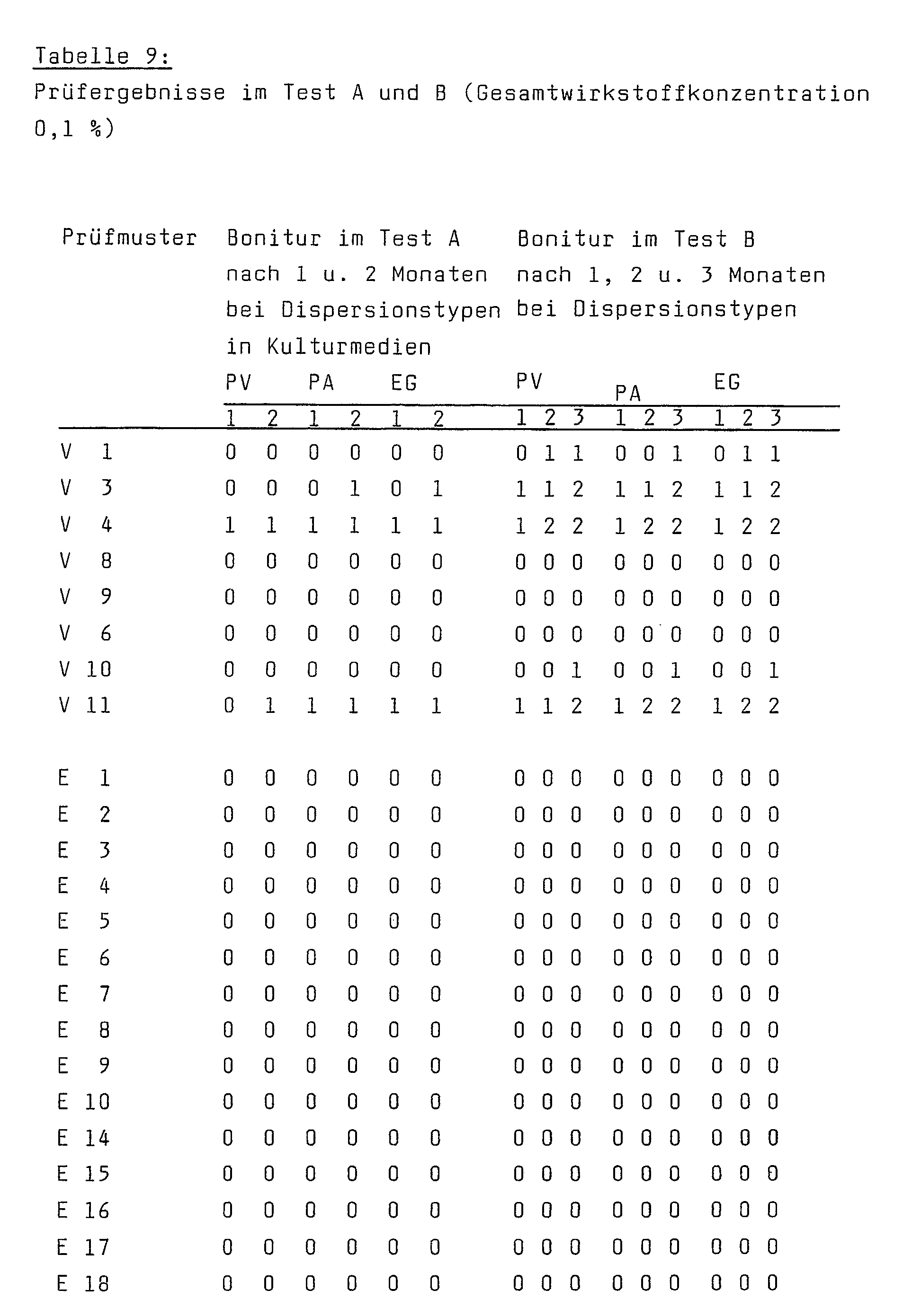

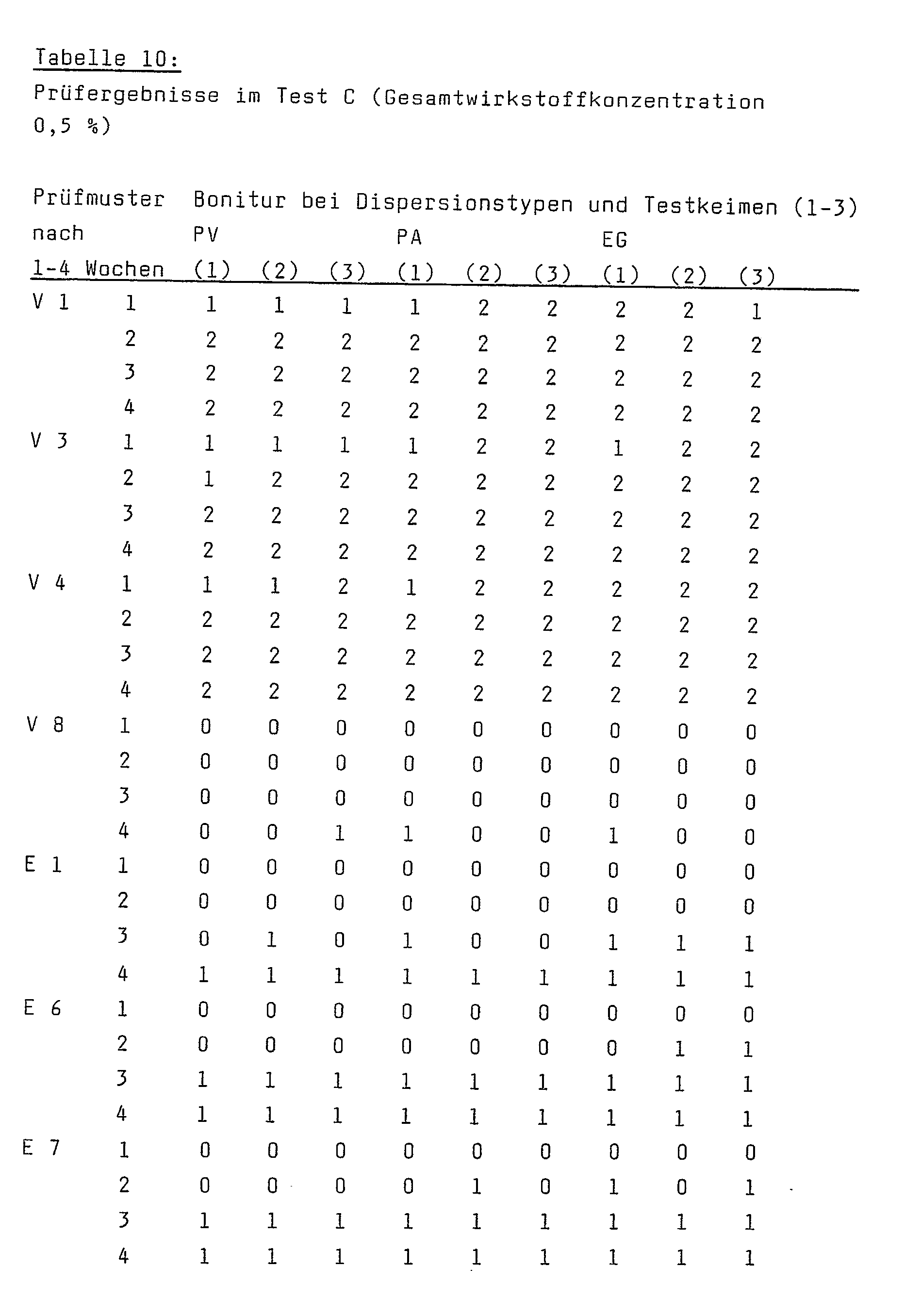

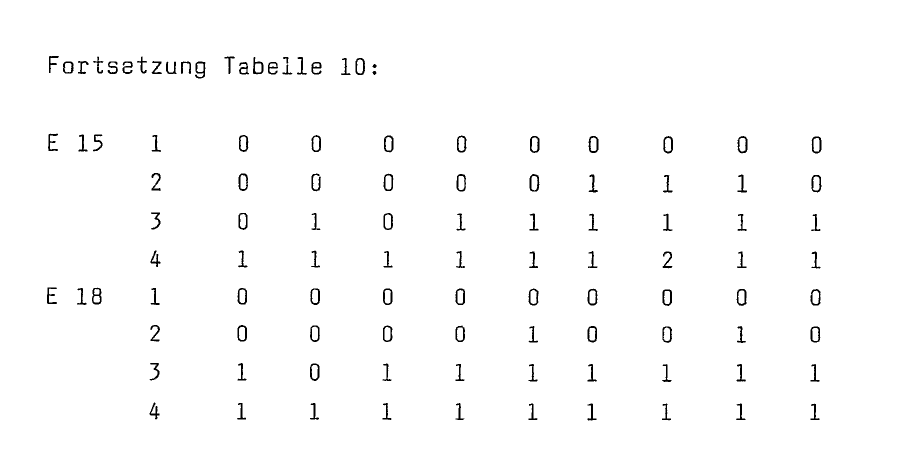

### 3.2.2. Haushaltchemikalien

### Prüfverfahren:

Den keimbelastenden Haushaltchemikalien (zwei Typen Geschirrspülmittel auf Basis von Tensidgemischen: S, D sowie ein Allzweckreiniger auf Basis von Tensidgemischen mit Harnstoffzusatz: A, im folgenden werden die Abkürzungen verwendet) wurden die Konservierungsmittel in bestimmten Konzentrationen (Angaben in % Gesamtwirkstoff) zugesetzt und nach einer Einwirkungszeit von 48 Stunden die Keimzahl pro ml Produkt durch Auszählen ermittelt. Die Ergebnisse sind in Tabellenform dargestellt.

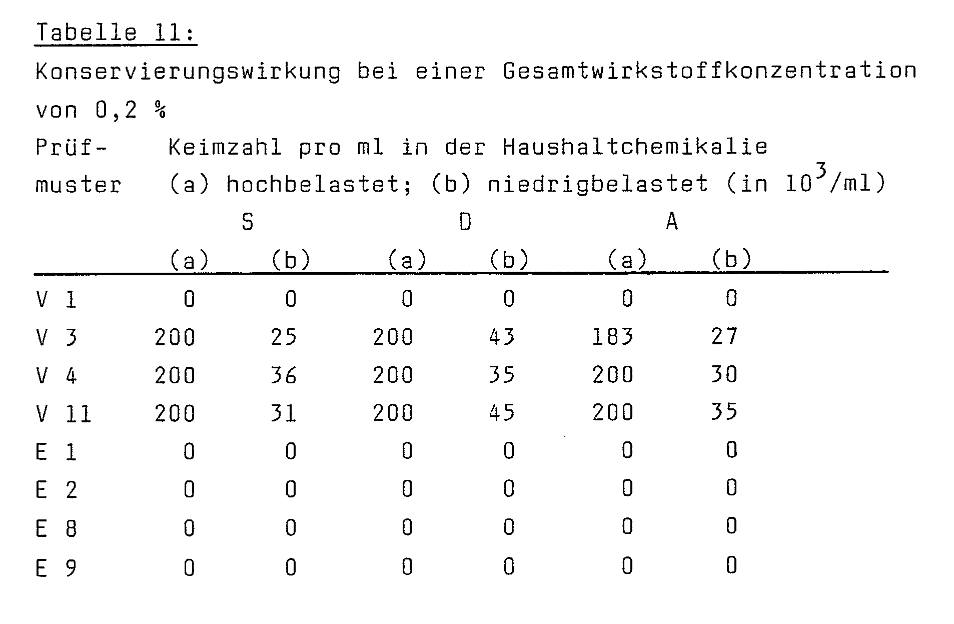

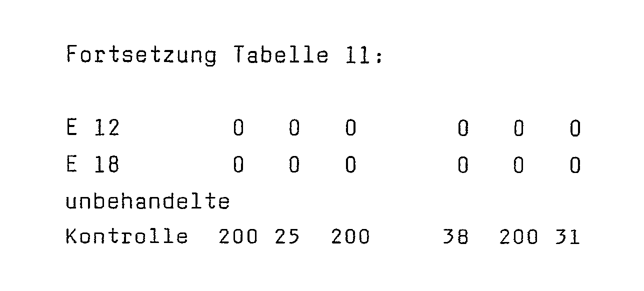

### 3.3 Anwendung als mikrobizide Ansätze für Kühlschmiermittel für die Metallbearbeitung

### Methodik:

Die Prüfung wurde unter praxisanalogen Bedingungen vorgenommen. Die Prüfdauer betrug 8 Wochen. Dazu wurden Bohrölemulsionen in Konzentrationen von 3 - 4,5 % eingesetzt, die durch Infektion mit typischen Erregern anfänglich Keimzahlen von 10⁶ - 10⁷ aufwiesen. Die Keimzahl wurde auf Nähragar mit Bakterienprüfröhrchen (VEB Feinchemie Sebnitz) bestimmt. Anerobe Bakterien wurden qualitativ durch H₂S-Bildung mit Bleiacetatpapier geprüft. Die Ergebnisse sind in Tabelle 12 dargestellt.

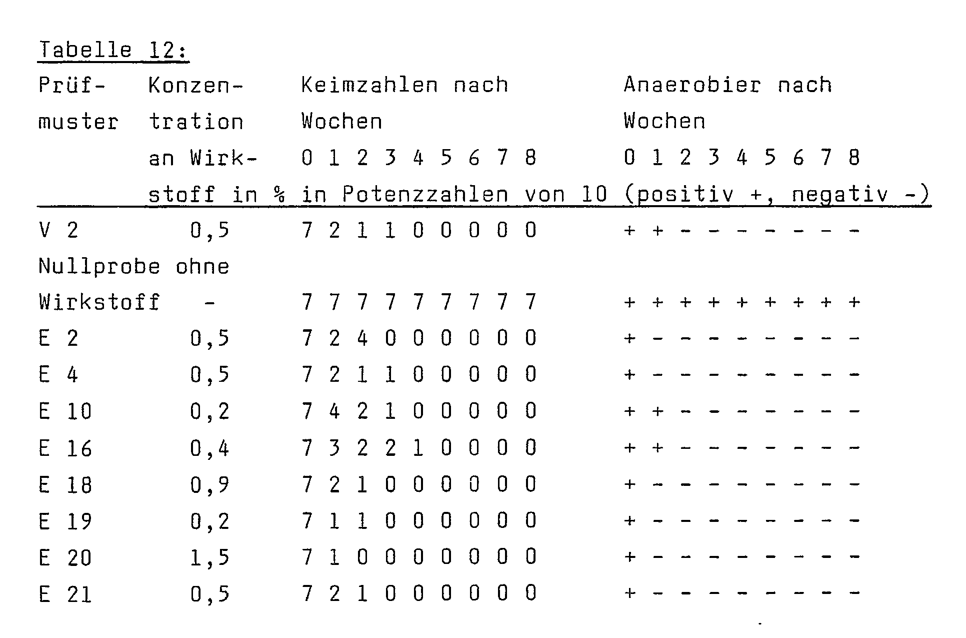

Des weiteren wurden Betriebsversuche mit analoger Kontrolluntersuchung durchgeführt. Das Umlaufsystem enthielt 200 1 Emulsion, alle 10 Tage wurden 100 1 zudosiert. Die Anwendungskonzentration betrug 0,5 %. Die Ergebnisse sind in Tabelle 13 dargestellt.

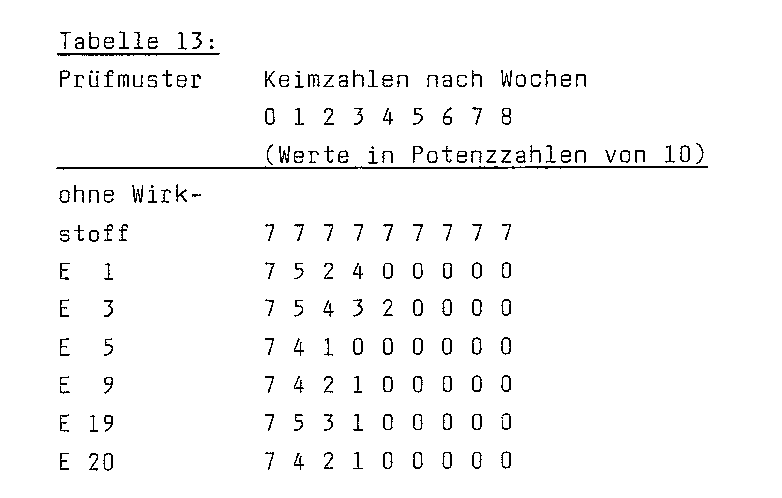

### Prüfung der Hautverträglichkeit:

Die Prüfung erfolgte tierexperimentell entsprechend der gesetzlichen Richtlinien dermal an weiblichen und männlichen Ratten vom Wistarstamm (Verfügungen und Mitteilungen des Ministeriums für Gesundheitswesen, Berlin 9/1986, S. 96). Die Wirkstoffe wurden 50 %ig in Wasser gelöst verwendet und die dermale LD₅₀ ermittelt. Die Ergebnisse sind in Tabellenform dargestellt.

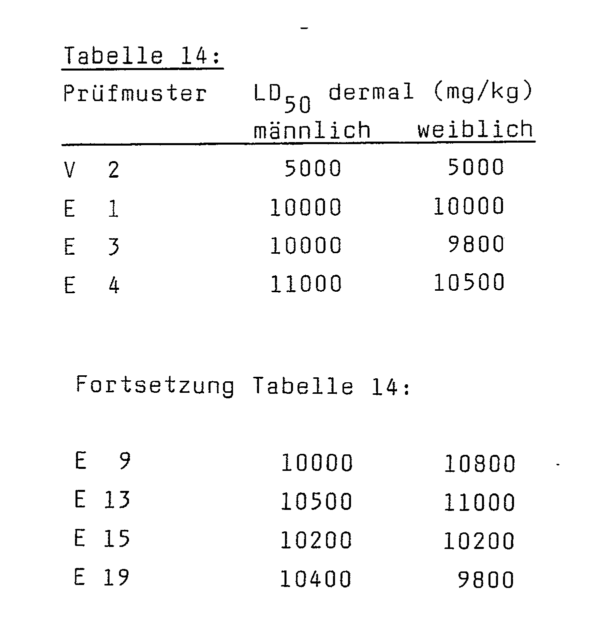

Die Praxiserprobung erfolgte in einer Metallbearbeitungsanlage. Dazu wurden erfindungsgemäße Mikrobizide (E 1, E 3 bzw. E 4) anstelle üblicher Konservierungsmittel 10 Wochen in Anwendungskonzentrationen von 0,5 % in 200 1 Kreislaufwasser angewendet, wobei nach jeder Woche ca. 100 1 Flüssigkeit neu zugegeben wurden. Dabei traten im Gegensatz zu Vergleichspräparaten keine Hautinitiationen auf.

### 3.4. Anwendung im Pflanzenschutz gegen Bakterien und Hefen

Die Vermehrungshemmung unter Einfluß der erfindungsgemäßen Mittel wurde im Flüssigkulturverfahren ermittelt. Einer Bakterien- bzw. Hefesuspension mit definierter Zellzahl wurden die Mittel bzw. Einzelbestandteile in den unten angegebenen Konzentrationen zugesetzt. Die Vermehrungshemmung wurde im optischen Meßverfahren über einen Zeitraum von 6 h ermittelt. Die Ergebnisse sind in der nachfolgenden Tabelle 15 dargestellt:

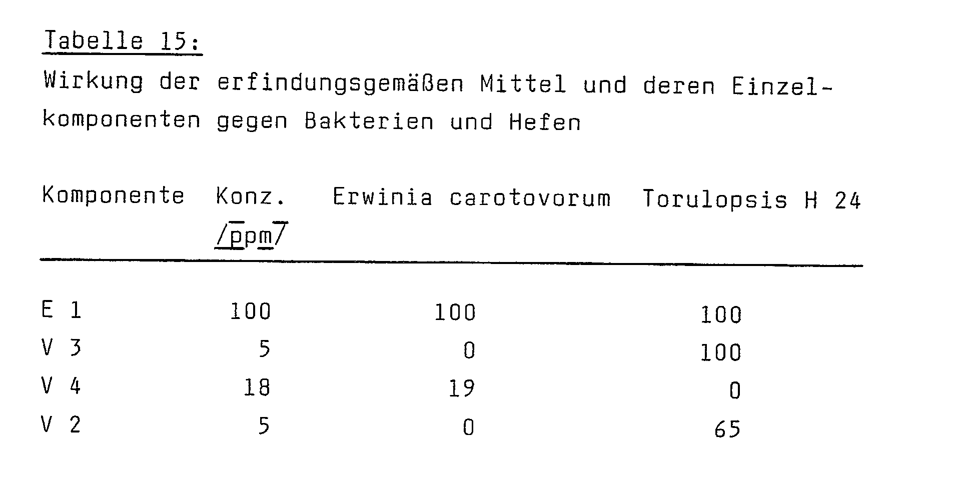

### Wirksamkeit der erfindungsgemäßen Pflanzenschutzmittel im Flüssigkulturverfahren

Die Prüfung von Vergleichsprodukten und erfindungsgemäßen Mittel erfolgte im Flüssigkulturverfahren, wobei die Konzentration in ppm ermittelt wurde, in dem Vermehrungshemmung (in %) erfolgt. Dazu wurden den Bakterien- bzw. Hefesuspensionen mit definierter Zellzahl die Prüfmuster in bestimmten Konzentrationen zugesetzt und die Vermehrungshemmung über einen Zeitraum von 6 Stunden quantitativ ermittelt.

Die Prüfergebnisse sind in Tabellenform dargestellt.

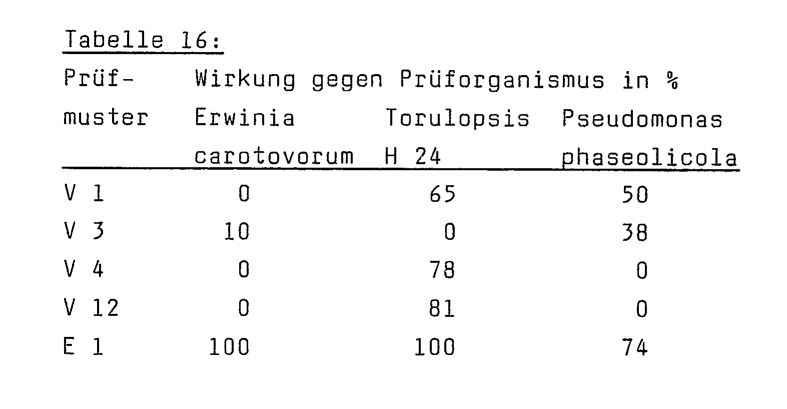

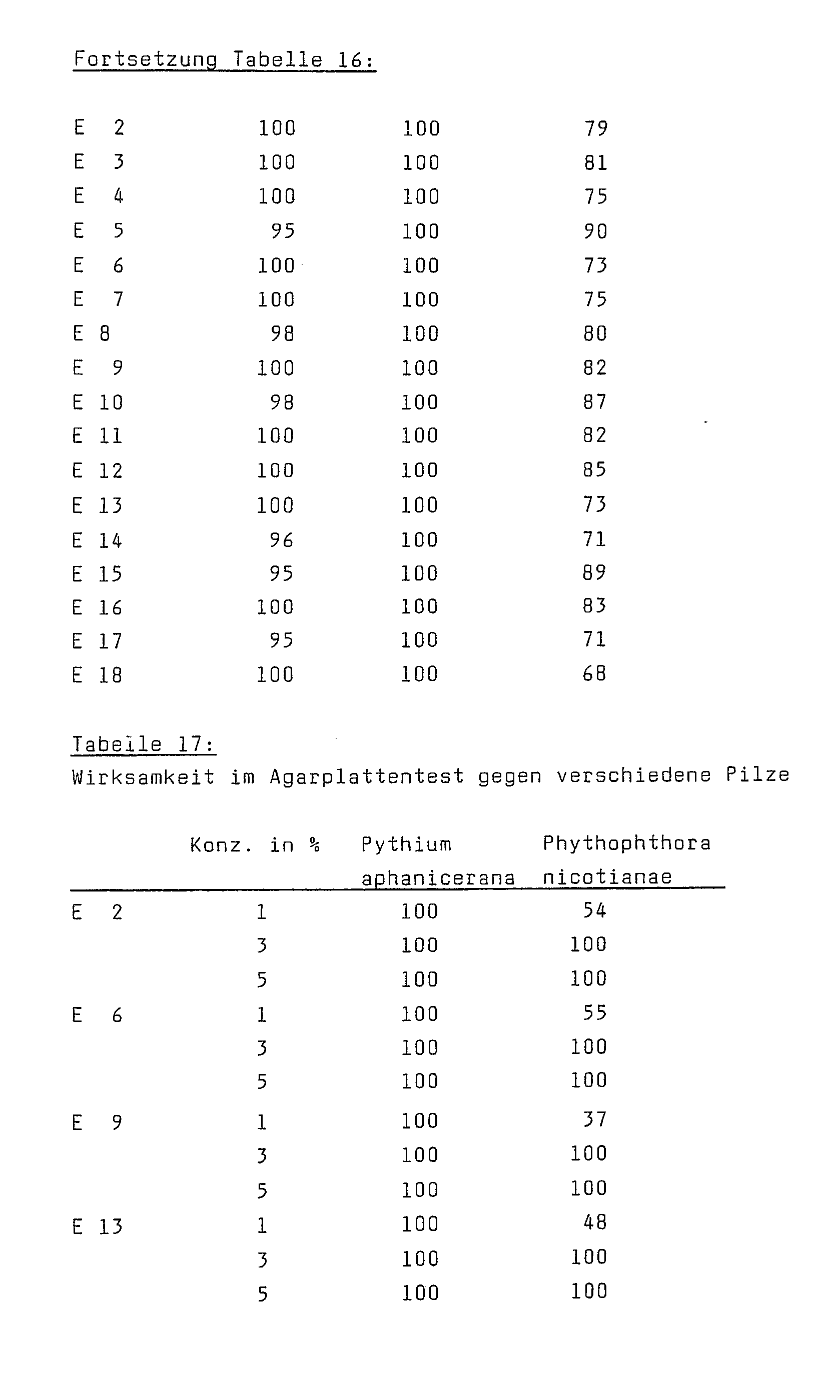

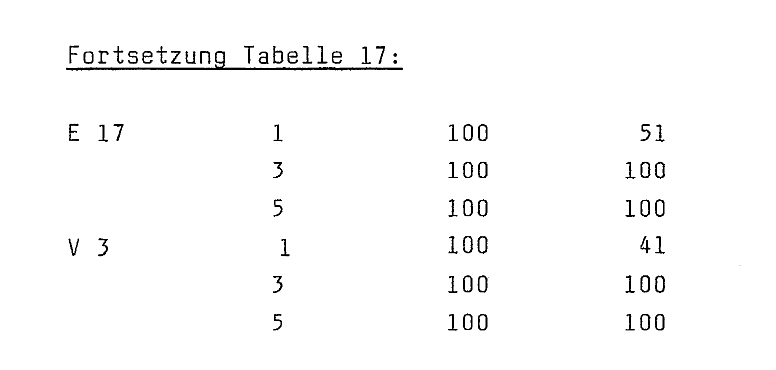

### Prüfung der Pflanzenverträglichkeit

Die Prüfung erfolgte durch Blatt- und Wurzelapplikation aus Methodik und Bonitur.

Die Blattapplikation wurde an getopften Pflanzen (Tomate, Gurke) durchgeführt. Dazu wurden die Pflanzen mit bestimmten Konzentrationen erfindungsgemäßer Mittel allseitig benetzt. Die Wurzelapplikation erfolgte an Pflanzen (Tomate, Gurke), die zunächst in Erde angezogen wurden. Danach wurden die Wurzeln mit Wasser gründlich ausgewaschen, zur Adaption 2 Tage in Wasser gesetzt und anschließend in wäßrige Lösungen mit bestimmten Konzentrationen erfindungsgemäßer Mittel mit und ohne Düngemittelzusatz (1 g/l Wopil) gebracht. Die Pflanzenverträglichkeit wurde 6 Tage nach der Blattapplikation bzw. 4 Tage nach der Wurzelapplikation anhand des folgenden Boniturschemas ermittelt:
4 = ohne Schäden - absolut pflanzenverträglich
3 = leichte Schäden - ohne Beeinträchtigung der weiteren
Entwicklung
2 = starke Schäden - mit Beeinträchtigung der weiteren
Entwicklung
1 = Pflanze total geschädigt - abgestorben
Die Ergebnisse sind in Tabellenform dargestellt:

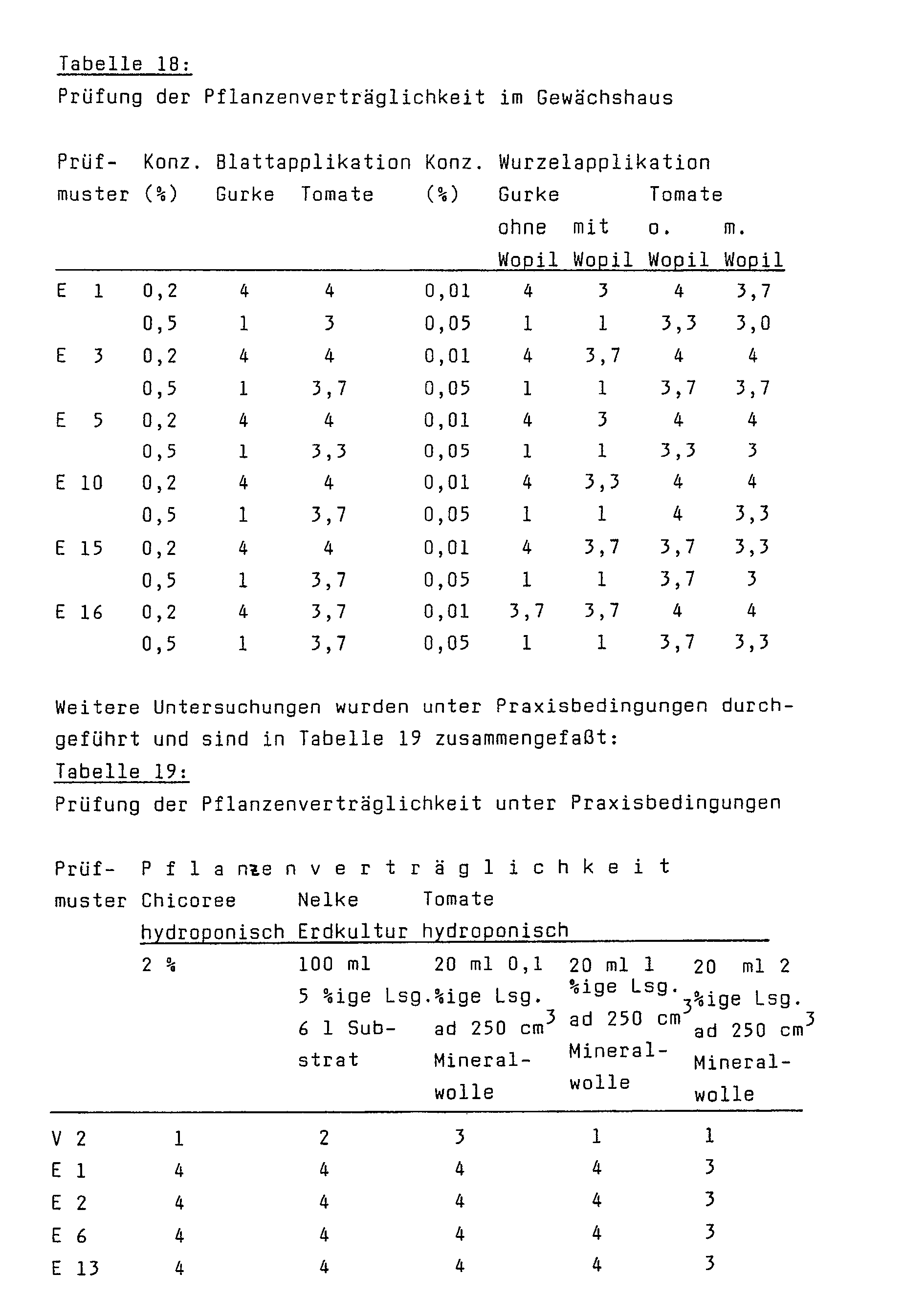

### Prüfung der Wirksamkeit als Desinfektionsmittel im Champignonanbau gegen Champignonmyzel

### Methodik:

Die Prüfung erfolgte in Anlehnung an das Verfahren von Lelley, I. (Der Champignon, 271 (1984), S. 14-24). Papierrundfilter wurden mittels Champignonsubstrat infiziert und in Petrischalen anschließend mit Vergleichsmustern und erfindungsgemäßen Konzentrationen in Kontakt gebracht. Nach 15-20 Minuten wurden die Filter entnommen und getrocknet. Die Filter wurden nun auf pasteurisiertes Substrat gelegt und 2 Tage bei 25 °C bebrütet. Danach erfolgt die Bonitur.
0 = kein Wachstum von Myzel
(1) = stellenweise schwaches Wachstum, gehemmt
1 = Wachstum von Myzel bis ins Substrat
2 = Wachstum auf der gesamten Oberfläche

Die Ergebnisse sind in einer Tabelle zusammengefaßt:

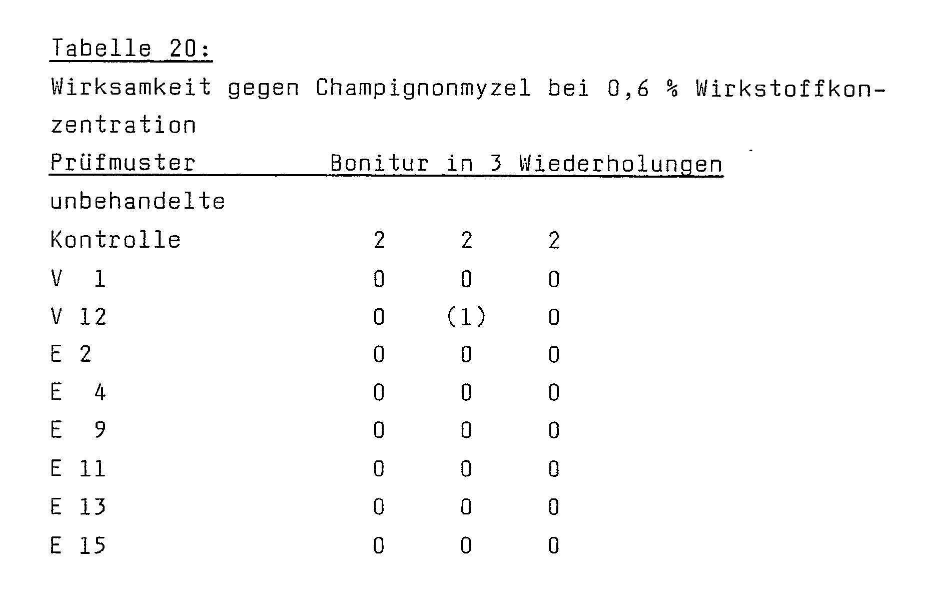

### 3.5. Wirkung der erfindungsgemäßen Pflanzenschutzmittel auf verschiedene Viren

Die Wirkung der erfindungsgemäßen Mittel wurde an folgenden Virus-Wirt-Kombinationen geprüft:
- Rotkleeschockungs-Virus (red dover mottle virus = RDMV) Erbse (Pisum sativum Diub. Alef "Nadja")
- Tabakmosaik-Virus (tobacco mosaic virus) Tabak (Nicotiana tabacum L. "Samsun NN")
- Kartoffel-Virus Y (potato virus Y = PVY) Tabak (Nicotiana tabacum L. "Samsun")
- Trespenmosaik-Virus (brome mosaik virus = BrMV) Wintergerste (Hordeum vulgare L. "Erfa") Mais (Zea mays L.)

Die Behandlung der Wirtspflanzen mit den Wirkstofflösungen erfolgte in der Regel 2 Tage vor und 2 Tage nach der Inokulation. Bei der Untersuchung der Wirkung gegenüber TMV wurden die Testpflanzen nur 2 Tage vorher behandelt. Eine zusätzliche Substanzapplikation sofort nach der Inokulation ist bei der Prüfung gegen TrMV, BMV und PVY erfolgt. Zur Anwendung kamen folgende Wirkstoffkonzentrationen: 0,25 %, 0,50 %, 0,75 % und in einem Fall auch 5,0 %. Am günstigsten erwiesen sich Konzentrationen von 0,25 % bis 0,5 %.

Zur Bestimmung der Virusgehalte wurden außer bei TMV nur serologische Methoden angewandt. Die Wirkung gegenüber TMV wurde durch die Reduzierung der Anzahl von Lokallösionen ermittelt.

Der RDMV-Gehalt in den Erbsenpflanzen wurde 8...9 Tage nach der Inokulation mittels Ringtest nach KLUGE und MARCINKA (1979) (Acta Virologicia 23, 148-152) bestimmt.

Zur Bestimmung von BrMV- und PVY-Gehalten in den Wirtspflanzen ist 14 Tage nach der Inokulation der ELISA durchgeführt worden. Außerdem wurde beim PVY der Virusgehalt in den inokulierten, also primär infizierten Blättern 7 Tage nach der Inokulation bestimmt. Zur Ermittlung der Hemmwirkung (in %) wurden Virusgehalte von mit Wasser behandelten Kontrollpflanzen zu denen mit Substanz behandelten Pflanzen ins Verhältnis gesetzt.
Die erfindungsgemäßen Mittel zeigen deutlich antivirale Wirkungen gegenüber schwer bekämpfbaren, ökonomisch bedeutsamen Viren von Kulturpflanzen. In der Tabelle sind die Viren-inhibierenden Effekte in Form der Hemmprozente ausgedrückt. Sie belegen eine Reduktion des Virusgehaltes im Durchschnitt zwischen 30 bis 40 %, im günstigsten Fall um 70 %.

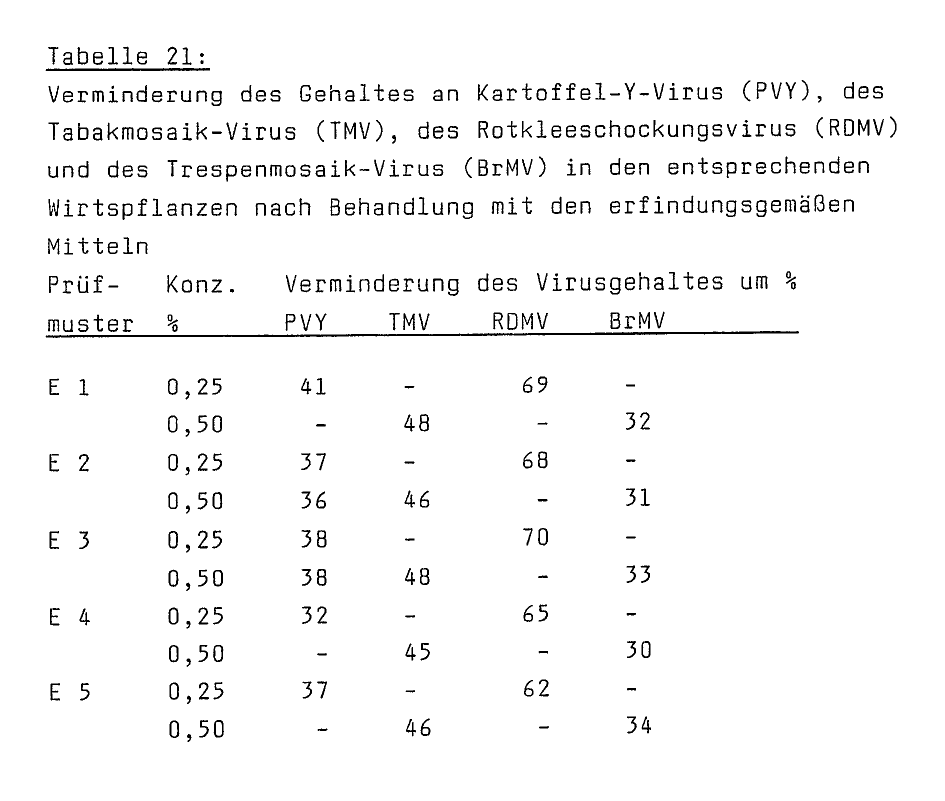

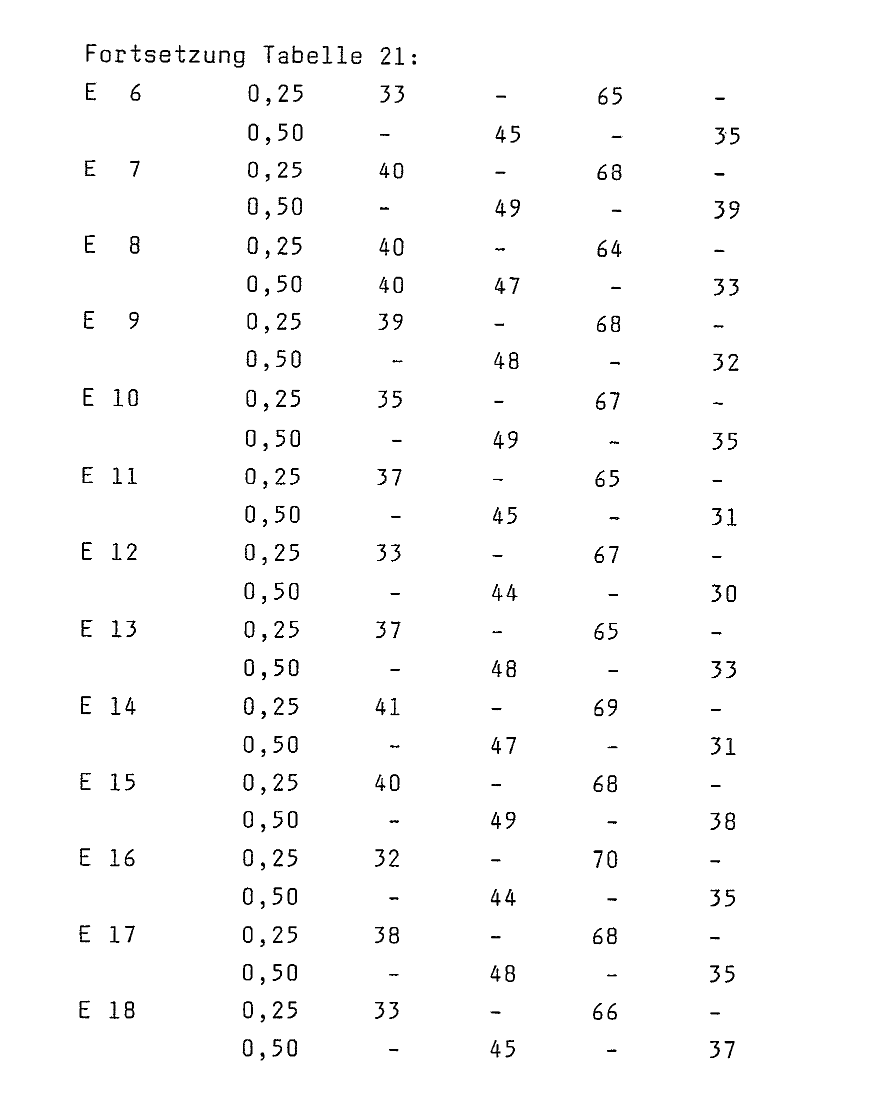

### 3.6. Anwendung als Zusätze für Flüssigwaschmittel

Vergleichsmuster und Zusammensetzung der erfindungsgemäßen Flüssigwaschmittel
Die Prüfungen von Vergleichswirkstoffen und erfindungsgemäßen neuen Wirkstoffen erfolgte in folgenden Rahmenrezepturen:

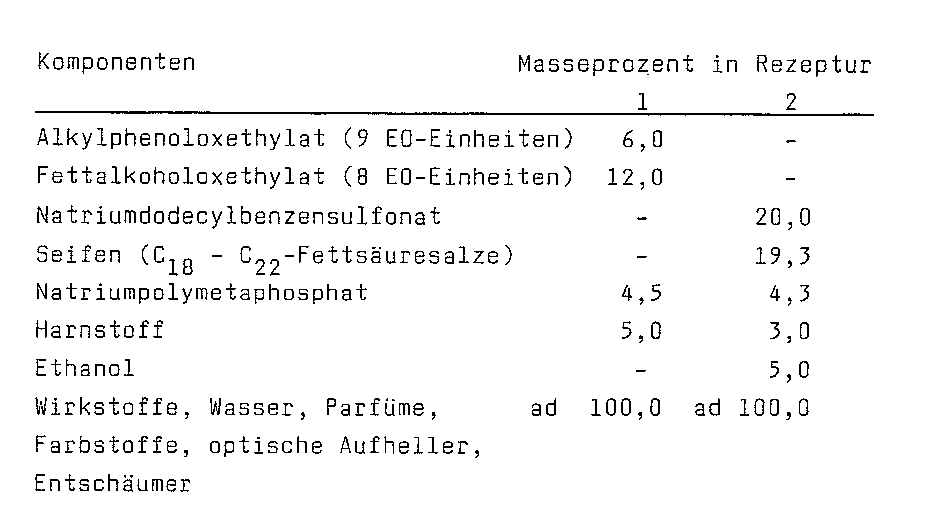

In diese Rahmenrezepturen wurden die zu prüfenden Wirkstoffe eingearbeitet. Als Vergleichsmuster wurde HHT (V 4) verwendet.

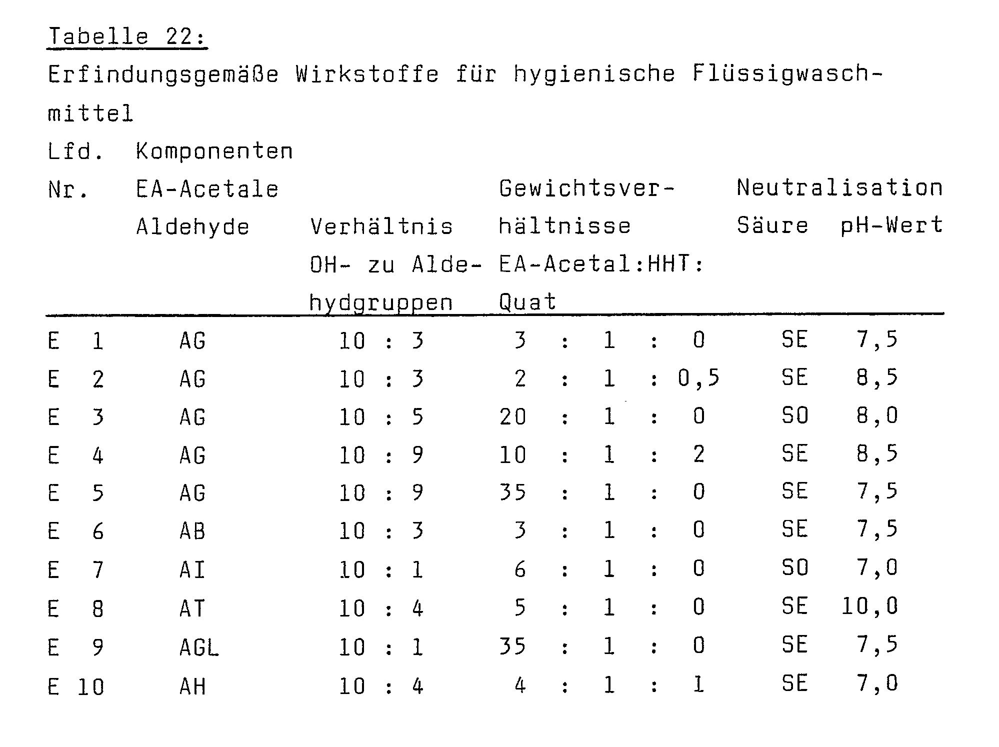

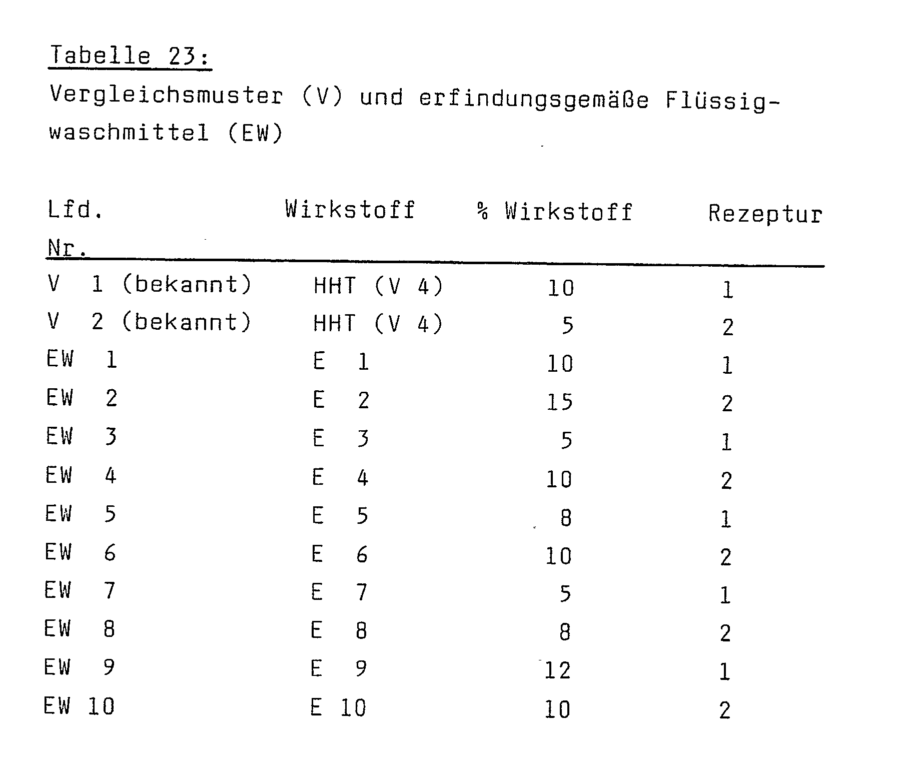

### Prüfung der antimikrobiellen Wirksamkeit von Flüssigwaschmitteln

### Methodik:

Die Testung der antimikrobiellen Wirksamkeit hygienischer Waschmittel erfolgte nach dem Prinzip der Testung von Desinfektionswaschverfahren. Das für diese Verfahren bewährte Testmodell wurde zu einer geeigneten praxisanalogen Testmethode variiert. Als Testkeime gelangten die folgenden Mirkoorganismen zum Einsatz:
- Staphylococcus aureus (1)
- Escherichia coli (2)
- Pseudomonas aeruginosa (3).

Die Vorbereitung der Testkeime erfolgte über jeweils 3 Passagen auf Nähragar, je Passage 24 Std. bei 37 °C.

Als Keimträger dienten sterile Leinenläppchen in den Größen 1 cm x 1 cm sowie 5 cm x 5 cm. Die Kontamination der Keimträger geschieht gemäß der folgenden Schritte:
- In Kolleschalen werden geeignete feste Nährmedien mit 16-stündigen Bouillonkulturen der Testkeime beimpft und anschließend 18 Stunden bei 37 °C bebrütet.
- Danach werden die Kolleschalen mit jeweils 100 ml steriler Magermilch abgespült.

In diese abgeschwemmte Keimsuspension werden die Keimträger für 15 min eingelegt und anschließend über Nacht bei Zimmertemperatur angetrocknet.
Die so vorbereiteten Keimträger werden der Wäsche mit hygienischen Waschmitteln ausgesetzt. Die praxisorientierte Versuchsanordnung besteht aus einem thermoregulierbaren Mehrhals-Schliffkolben, in dem die erforderliche Bewegung des Waschgutes mit Hilfe eines Rührwerkes gesichert ist. Durch entsprechende sterile Zusatzvorrichtungen wird gewährleistet, daß eine kontaminationsverhindernde Beschickung der Waschflotte mit den Keimträgern möglich ist. Das gilt gleichermaßen für die Entnahme der Proben nach der hygienischen Waschbehandlung, wodurch Fehlergebnisse zu vermeiden sind. Nach dem hygienischen Waschvorgang (30 min bei 30 °C) werden die Testläppchen unter sterilen Bedingungen entnommen und 2 mal mit sterilem Wasser gespült, wobei dem ersten Spülwasser 0,5 % Cystein als Inaktivatorsubstanz zugesetzt wurde.
Die kleinen Testläppchen werden sodann in 10 ml Nährbouillon eingelegt und für 5 Tage bei 37 °C bebrütet. Die Testläppchen der Größe 5 cm x 5 cm werden auf geeignete feste Nährmedien aufgelegt, dort für 2 Std. belassen und anschließend entfernt. Die Nährmedien werden 5 Tage bei 37 °C bebrütet. Den Nährmedien werden spezifische Inaktivatoren zugesetzt. Ausgewertet wird sowohl quantitativ durch Auszählen der gewachsenen Kolonien (große Testläppchen) bzw. durch Anlegen von Zählplatten (Ausspateln von 0,1 und 1 ml der flüssigen Nährmedien - kleine Testläppchen) wie auch qualitativ durch Feststellen des Bewuchses der Nährmedien.

Als Kontrolle dienten kontaminierte Keimträger, die nicht der Behandlung in hygienischen Waschverfahren ausgesetzt waren.

### Ergebnisse

Die Bewertung der keimzahlreduzierenden Waschleistung erfolgte nach folgendem Schema:
3 = sehr gute keimzahlreduzierende Wirkung
2 = ausreichende keimzahlreduzierende Wirkung
1 = schwache keimzahlreduzierende Wirkung
0 = keine keimzahlreduzierende Wirkung

Die Ergebnisse der Prüfungen bei 30, 40, 50 und 60 °C sind in der folgenden Tabelle 24 dargestellt:

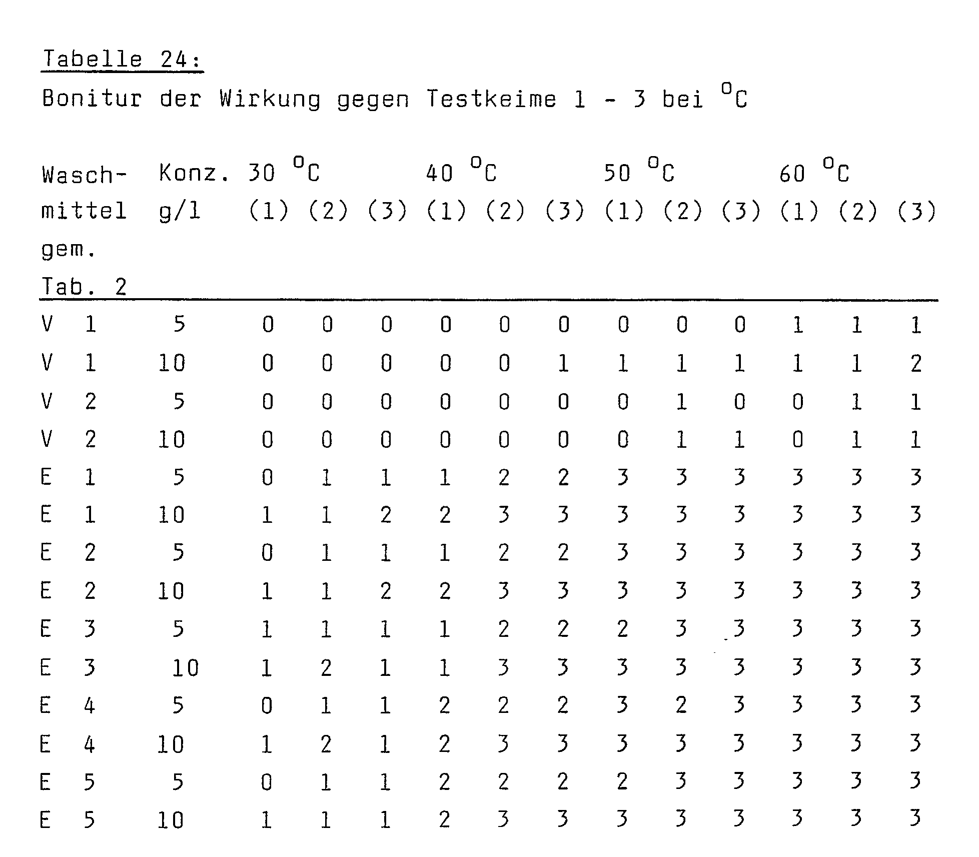

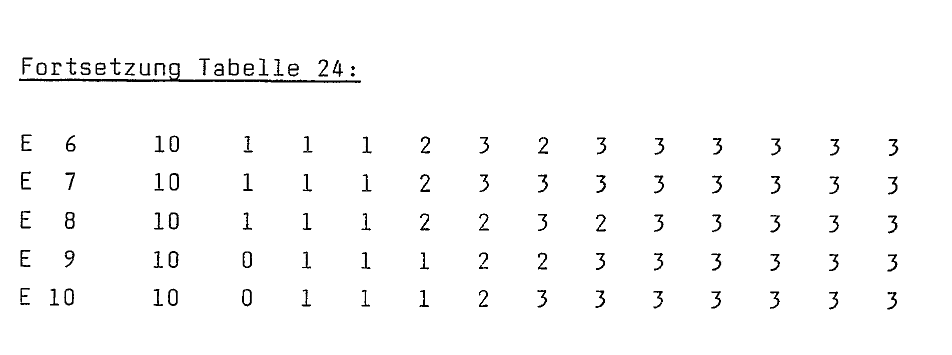

### Prüfung des Waschvermögens:

Die Prüfung des Waschvermögens von Mustern gemäß Beispiel Tabelle 23 erfolgte im Vergleich zu im Handel befindlichen flüssigen Feinwaschmitteln (F 1, F 2).

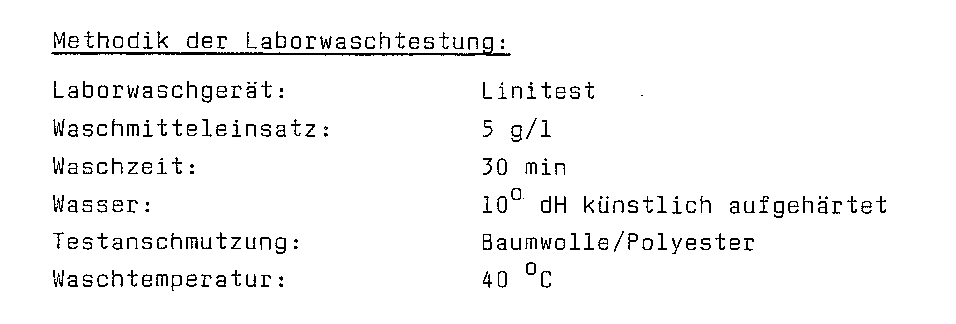

Die Ergebnisse werden in % Waschvermögen ermittelt. Sie sind in Tabelle 25 zusammengefaßt dargestellt.

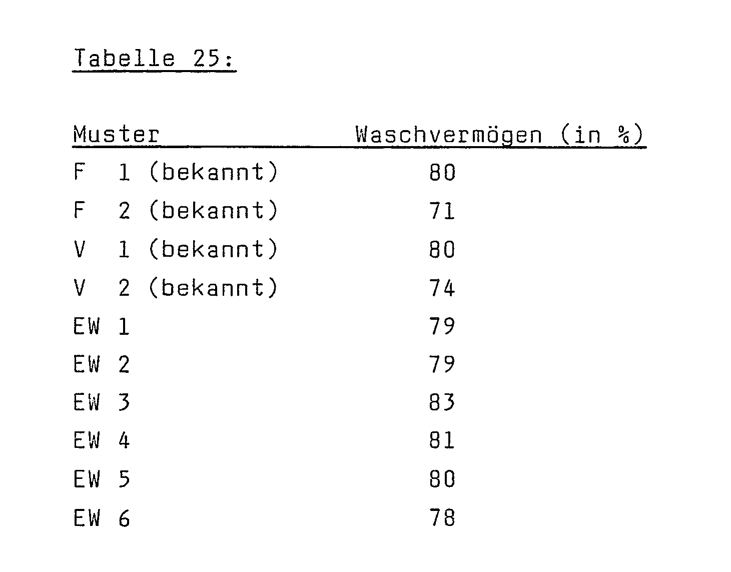

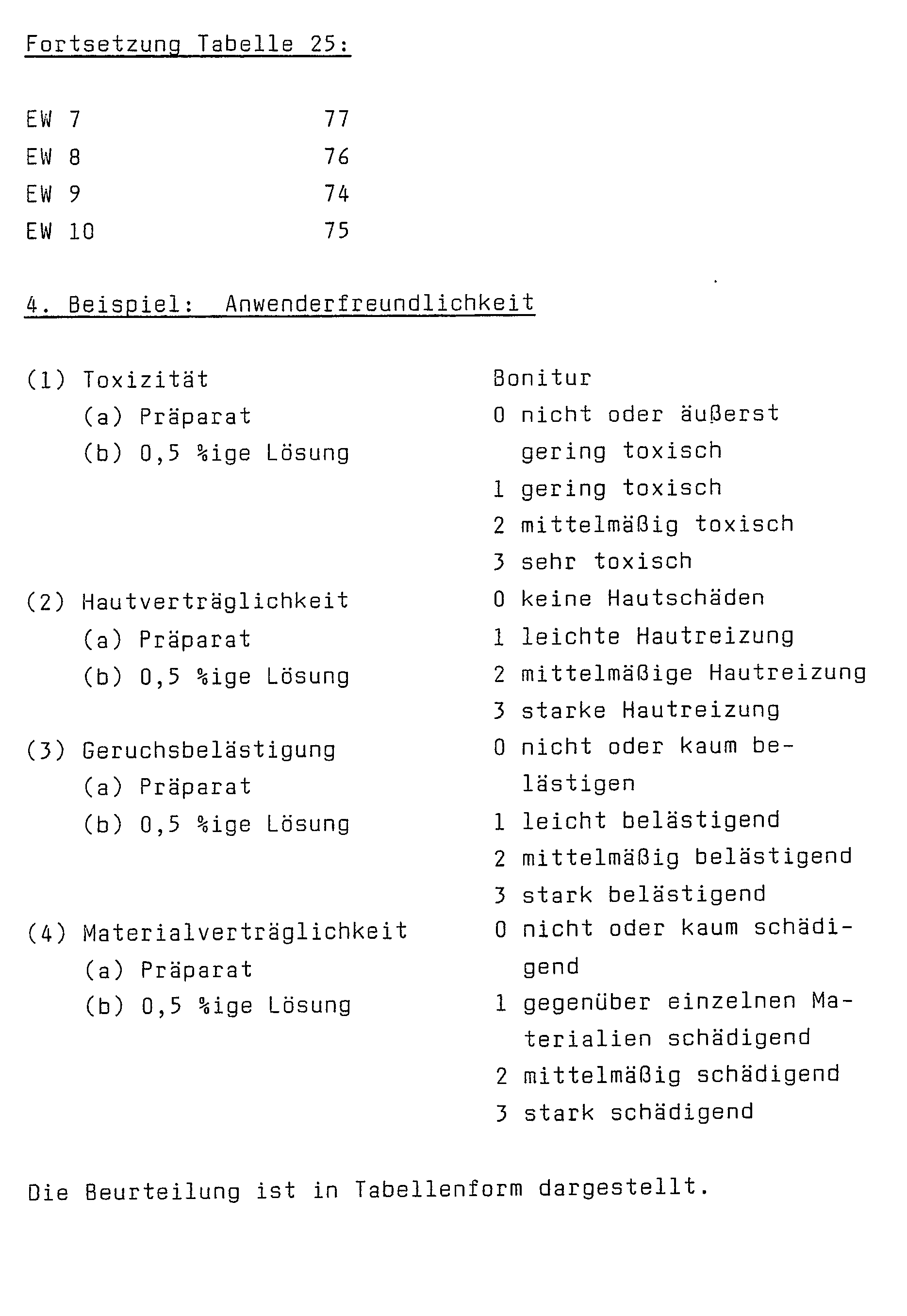

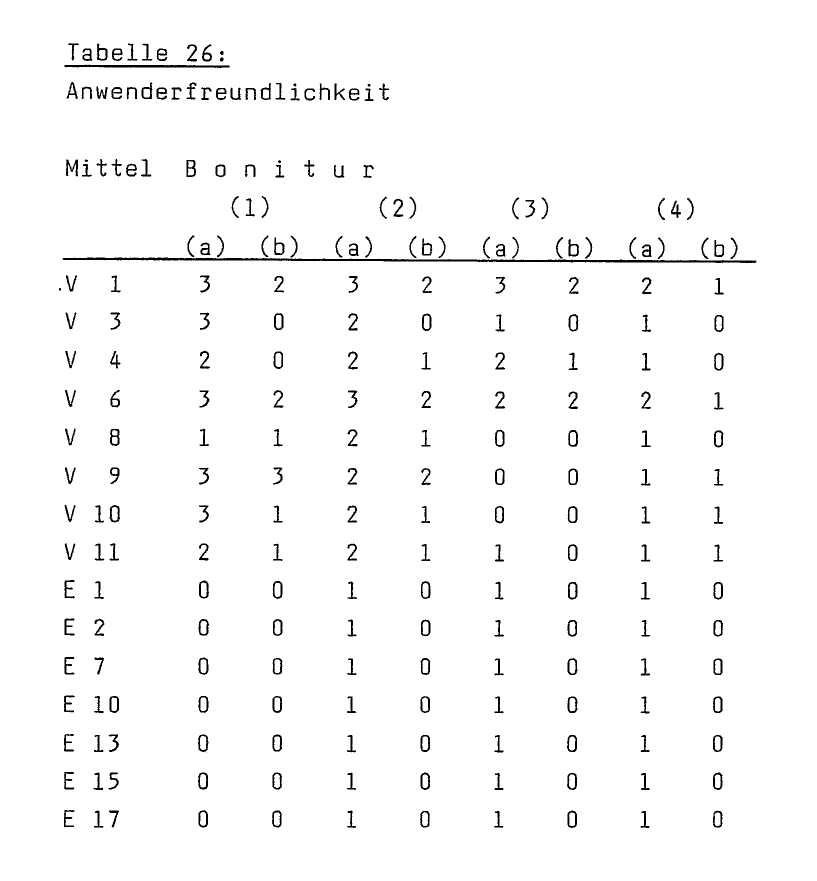

## Patentansprüche

1. Bakterizide, virizide und fungizide Mischung auf Basis von lagerstabilen acetalartigen Wirkstoffen und bekannten Hilfs- und Trägerstoffen, gekennzeichnet dadurch, daß die Wirkstoffe durch Umsetzung von 1,3,5-Tris-(2-hydroxyethyl)-hexahydrotriazin oder äquimolaren Mengen Formaldehyd und Ethanolamin mit aliphatischen oder aromatischen Mono- oder Dialdehyden bei 10 °C bis 100 °C mit einem Äquivalenzverhältnis der OH-Gruppen zu den Aldehydgruppen von 10 : 1 bis 9 entstehen.

2. Bakterizide, virizide und fungizide Mischung nach Anspruch 1, gekennzeichnet dadurch, daß bei der Herstellung dieser Verbindungen als aliphatische oder aromatische Mono- oder Dialdehyde vorzugsweise solche mit 2 bis 8 C-Atomen, beispielsweise Glutaraldehyd, Malondialdehyd, n- oder iso-Butyraldehyd, Propionaldehyd, Acetaldehyd, Chloral, Benzaldehyd oder Vinillin verwendet werden.

3. Bakterizide, virizide und fungizide Mischung nach Ansprüchen 1 und 2, gekennzeichnet dadurch, daß bei der Herstellung dieser Verbindungen als Lösungsmittel vorzugsweise Wasser, Alkohole, Ether, chlorierte Kohlenwasserstoffe oder Aromaten verwendet werden.

4. Bakterizide, virizide und fungizide Mischung nach Ansprüchen 1, 2 und 3, gekennzeichnet dadurch, daß bei der Herstellung dieser Verbindungen nach der Umsetzung ein pH-Wert von 4 bis 12 durch Zugabe organischer oder anorganischer Säuren eingestellt werden kann.

5. Verwendung der bakteriziden, viriziden und fungiziden Mischung nach Ansprüchen 1 bis 4 als anwenderfreundliche Konservierungs- und Desinfektionsmittel, gekennzeichnet dadurch, daß die Wirkstoffe neben bekannten mikrobizid wirksamen Verbindungen und 1,3,5-Tris-(2-hydroxyethyl)-hexahydrotriazin im Gewichtsverhältnis von 0,5 bis 20 : 0,1 bis 10 : 1 enthalten sind.

6. Verwendung der bakteriziden, viriziden und fungiziden Mischung als wasserlösliche Pflanzenschutzmittel zur Bekämpfung von Bakterien, Pilzen oder Viren nach Ansprüchen 1 bis 4, gekennzeichnet dadurch, daß die Wirkstoffe neben bekannten quartären mikrobizid wirksamen Ammoniumsalzen und 1,3,5-Tris-(2-hydroxyethyl)-hexahydrotriazin im Gewichtsverhältnis von 0,5 bis 20 : 0,1 bis 10 : 1 enthalten sind.

7. Verwendung der bakteriziden, viriziden und fungiziden Mischung als Zusatz zu hygienisch wirkenden Flüssigwaschmitteln nach Ansprüchen 1 bis 4,gekennzeichnet dadurch, daß die Wirkstoffe neben quartären Ammoniumverbindungen und 1,3,5-Tris-(2-hydroxyethyl)-hexahydrotriazin im Gewichtsverhältnis von 0,5 bis 20 : 0,5 bis 10 : 1 enthalten sind.

8. Verwendung der bakteriziden, viriziden und fungiziden Mischung als mikrobizide Zusätze für Kühlschmiermittel der Metallbearbeitung nach Ansprüchen 1 bis 4, gekennzeichnet dadurch, daß die Wirkstoffe neben bekannten quartären mikrobizid wirksamen Verbindungen und 1,3,5-Tris-(2-hydroxyethyl)-hexahydrotriazin im Gewichtsverhältnis von 0,5 bis 20 : 0,1 bis 10 : 1 enthalten sind.
